# EUROPEAN PATENT APPLICATION

(11) **EP 4 166 654 A1**
(43) Date of publication of application: **19.04.2023**
(21) Application number: 21825053.8
(22) Date of filing: 28.05.2021
(51) Int. Cl.: C12N 5/0775, A61L 27/24, A61L 27/38, C12M 1/00, C12N 5/074

(54) **SOMATIC STEM CELL INTEGRATED TISSUE STRUCTURE AND APPARATUS FOR PRODUCING SAME**

(30) Priority: 16.06.2020 JP 2020103747
(71) Applicant: Biotube Co., Ltd., Tokyo, 104-0033 (JP)
(72) Inventor: NAKAYAMA Yasuhide, Tokyo 104-0033 (JP); SATO Yasushi, Tokyo 104-0033 (JP); IWAI Ryosuke, Okayama-shi, Okayama 700-0005 (JP); FUKUSHIMA Masanori, Nagoya-shi, Aichi 458-0045 (JP); TAKENAKA Hiroshi, Kyoto-shi, Kyoto 606-0855 (JP)
(74) Representative: J A Kemp LLP
(86) International application number: PCT/JP2021/020347
(87) International publication number: WO 2021/256216

(57) **Abstract**

This invention relates to a tissue construct comprising a core portion having a recess and composed of fibrous connective tissue, and loose fibrous somatic stem cell-accumulated tissue comprising type III collagen and somatic stem cells which is formed in the recess; a device for producing the same; and a method for collecting somatic stem cells from the tissue construct.

## Description

### Technical Field

The present invention relates to a somatic stem cell-accumulated tissue construct and a device for producing the same.

### Background Art

The self-defense reaction to eliminate foreign substances causes macrophages and the like to be accumulated around foreign substances that have invaded into a living body. Macrophages adsorb to the surfaces of the foreign substances to degrade foreign substances, and promote collagen production in fibroblasts through TGF-β production by monocytes. As a result, the foreign substances that have invaded into a living body are covered by connective tissues comprising fibroblasts and collagen to be isolated in a living body (i.e., encapsulation). As a technology for forming living body-derived tissue on the basis of such phenomena, a technique of forming connective tissue by implanting a base material, which is a foreign substance, in a living body is reported (e.g., Patent Documents 1 to 6). For example, Patent Document 5 discloses that a shell member formed helically along the circumference of the rod-like structural member is implanted in a living body, thereby producing a connective tissue body formed on the surface of the rod-like structural member and including the shell member embedded therein. Patent Document 6 discloses that an assembly comprising a rod-like body and a stent fitted on the circumferential surface of the rod-like body is implanted in a living body to form a connective tissue body on the circumferential surface of the assembly, the resultant is isolated from the living body, and the rod-like body is removed therefrom, thereby obtaining a stent covered by the connective tissue body. However, the tissue formed on the surface of the base material via encapsulation generally has a thickness of only approximately several tens to several hundreds of micrometers, and most of the cells included therein are fibroblasts.

In recent years, regenerative therapies by implanting cells cultured *ex vivo* or tissue constructed therefrom in a living body have drawn attention as a technology for reproducing functions of tissues or organs impaired by diseases or injuries. Stem cells are important materials for production of cells of interest or construction of tissues in regenerative therapies. Meanwhile, the basis for the use in regenerative therapies of embryonic stem cells that are required to be ethically considered, or of induced pluripotent stem cells of which safety is required to be assured has not been yet established. Accordingly, a technique of efficiently collecting somatic stem cells that are safer and less problematic in terms of ethics has been awaited.

### Citation List

### Patent Documents

Patent Document 1: JP 2007-312821 A
Patent Document 2: JP 2008-237896 A
Patent Document 3: JP 2010-094476 A
Patent Document 4: JP 2012-105860 A
Patent Document 5: WO 2016/076416
Patent Document 6: JP 2006-255288 A

### Summary of the Invention

### Problem to Be Solved by the Invention

An object of the present invention is to provide a technique of efficiently collecting somatic stem cells.

### Means for Solution to Problem

The present inventors have conducted concentrated studies in order to solve the above problem. As a result, they found that a tissue construct comprising somatic stem cells accumulated therein could be produced. This has led to the completion of the present invention.

Specifically, the present invention includes the following.
[1] A tissue construct comprising a core portion having a recess and composed of fibrous connective tissue, and loose fibrous, somatic stem cell-accumulated tissue comprising type III collagen and somatic stem cells which is formed in the recess.
[2] The tissue construct according to [1] above, wherein the core portion has one, or two or more recesses.
[3] The tissue construct according to [1] or [2] above, wherein the core portion has at least three recesses.
[4] The tissue construct according to any of [1] to [3] above, wherein the recess has an opening width of at least 2.5 mm.
[5] The tissue construct according to any of [1] to [4] above, wherein the core portion has a diametrical dimension of at least 2.5 mm.
[6] The tissue construct according to any of [1] to [5] above, wherein the somatic stem cells comprise at least either mesenchymal stem cells or pluripotent stem cells.
[7] The tissue construct according to any of [1] to [6] above, wherein the tissue construct has a rod-like shape, and a plurality of the recesses are located along the circumferential direction.
[8] The tissue construct according to any of [1] to [7] above, wherein the tissue construct has a rod-like shape, and a plurality of the recesses are located along the axial direction.
[9] The tissue construct according to any of [1] to [6] above, wherein the tissue construct has a generally polyhedral shape, and a plurality of the recesses are located on different planes thereof.
[10] The tissue construct according to any of [1] to [9] above, wherein the tissue construct has a non-tubular and non-sheet shape.
[11] The tissue construct according to any of [1] to [10] above, which is formed by placing a device with a hollow portion for producing a tissue construct having a hollow portion in an environment comprising body tissue materials,
   wherein the device for producing a tissue construct has a frame body that forms the hollow portion,
   the frame body has an opening that communicates from the hollow portion to an external space of the device for producing a tissue construct, and
   the frame body defines the shape of the tissue construct formed in the hollow portion.
[12] The tissue construct according to [11] above, wherein the tissue construct fills the hollow portion in the device for producing a tissue construct and the core portion is adhered to the surface of the frame body.
[13] A method for collecting somatic stem cells comprising separating somatic stem cell-accumulated tissue or somatic stem cells from the tissue construct according to any of [1] to [12] above.
[14] A device for producing a tissue construct comprising:
   a hollow portion; and
   a frame body that forms the hollow portion,
      wherein the frame body has an opening that communicates from the hollow portion to an external space of the device for producing a tissue construct, and
      the tissue construct comprises a core portion having a recess and composed of fibrous connective tissue, and loose fibrous, somatic stem cell-accumulated tissue comprising type III collagen and somatic stem cells which is formed in the recess.
[15] The device for producing a tissue construct according to [14] above, which is used to form a tissue construct comprising a core portion having a recess and composed of fibrous connective tissue, and loose fibrous, somatic stem cell-accumulated tissue comprising type III collagen and somatic stem cells which is formed in the recess by placing the device in an environment comprising body tissue materials,
   wherein the device for producing a tissue construct comprises a hollow portion, and a frame body that forms the hollow portion,
   the frame body has an opening that communicates from the hollow portion to an external space of the device for producing a tissue construct,
   the frame body defines the shape of the tissue construct formed in the hollow portion, and
   when the device is placed in the environment comprising body tissue materials, fibrous connective tissue is formed to stretch from the surface of the frame body toward the inside of the hollow portion, thereby causing the core portion having a recess recessed from the opening toward the hollow portion to be formed, and loose fibrous tissue where somatic stem cells are accumulated is formed in the recess.
[16] The device for producing a tissue construct according to [14] or [15] above, wherein the opening has an opening width of at least 2.5 mm.
[17] The device for producing a tissue construct according to any of [14] to [16] above, wherein the opening has an opening configuration that enables a circle with a diameter of 2.5 mm or larger to be inscribed therein.
[18] The device for producing a tissue construct according to any of [14] to [17] above, wherein the frame body is composed of a columnar member and a supporting member, and the columnar member is fixed by the supporting member to maintain the shape of the frame body.
[19] The device for producing a tissue construct according to [18] above, wherein a plurality of the columnar members are fixed to the supporting members at the both ends of the columnar members and arranged in a circumferential direction on a hypothetical cylindrical plane sandwiched between the supporting members.
[20] The device for producing a tissue construct according to [18] or [19] above, wherein the opening is formed by the columnar member and the supporting member, or a plurality of the columnar members.
[21] The device for producing a tissue construct according to any of [14] to [20] above, wherein the opening has a polygonal, rectangular, trapezoidal, round, circular, or elliptic opening edge.
[22] The device for producing a tissue construct according to any of [18] to [21] above, wherein the supporting member further has an opening that communicates from the hollow portion to an external space of the device.
[23] A method for producing a tissue construct comprising a core portion having a recess and composed of fibrous connective tissue, and loose fibrous somatic stem cell-accumulated tissue comprising type III collagen and somatic stem cells which is formed in the recess,
   wherein the method comprises placing the device for producing a tissue construct according to any of [14] to [22] above in an environment comprising body tissue materials, such as in a living body or in an isolated body tissue.

This description includes the contents as disclosed in Japanese Patent Application No. 2020-103747 from which the present application claims priority.

### Effects of the Invention

According to the present invention, somatic stem cells can be collected efficiently.

### Brief Description of the Drawings

[Fig. 1] Fig. 1 is an oblique view showing an oblique perspective structure of an embodiment of a device for producing a tissue construct.
[Fig. 2] Fig. 2 is a cross-sectional view showing a cross-sectional structure of an embodiment of a device for producing a tissue construct.
[Fig. 3] Fig. 3 presents schematic diagrams schematically illustrating how somatic stem cells are accumulated with the use of a device for producing a tissue construct in sequential order from A to D.
[Fig. 4] Figs. 4A to 4D present an oblique view showing an oblique perspective structure of an embodiment of a device for producing a tissue construct.
[Fig. 5] Figs. 5A and 5B present an oblique view showing an oblique perspective structure of an embodiment of a device for producing a tissue construct according to an embodiment.
[Fig. 6] Figs. 6A to 6C present an oblique view showing an oblique perspective structure of an embodiment of a device for producing a tissue construct according to an embodiment.
[Fig. 7] Figs. 7 presents an oblique view showing an oblique perspective structure of a modified example of a device for producing a tissue construct according to a modification example.
[Fig. 8] Fig. 8 presents an oblique view showing an oblique perspective structure of a modified example of a device for producing a tissue construct according to a modification example.
[Fig. 9] Fig. 9 presents an oblique view showing an oblique perspective structure of a modified example of a device for producing a tissue construct according to a modification example.
[Fig. 10] Fig. 10 presents photographs showing the results of the production test of a tissue construct comprising somatic stem cells accumulated therein with the use of a device for producing a tissue construct. A, a device as used; B, a device comprising a tissue construct formed therein, which has been removed after implantation in a living body; C, a cross section of the resulting tissue construct.
[Fig. 11] Fig. 11 presents photographs showing the results of the staining test of the tissue construct. A, HE staining; B, MT staining; C, EVG staining; D: SR staining.
[Fig. 12] Fig. 12 presents polarizing micrographs of sections stained with Sirius Red. A, around a columnar member; B, center part; C, between columnar members.
[Fig. 13] Fig. 13 presents photographs showing the results of immunohistochemical staining of the tissue construct for mesenchymal stem cell markers CD90 and CD105. Left column, CD90/DAPI; right column, CD105/DAPI; A and B, pocket portions; C and D, around a columnar member; E and F, center part.
[Fig. 14] Fig. 14 presents photographs showing the results of immunohistochemical staining of the tissue construct for pluripotent stem cell markers SSEA4 and SSEA3. Left column, SSEA4/DAPI; right column, SSEA3/DAPI; A and B, pocket portions; C and D, around a columnar member; E and F, center part.
[Fig. 15] Fig. 15 presents photographs showing the results of immunohistochemical staining of the tissue construct for growth factors: vascular endothelial growth factors (VEGF) and hepatic cell growth factors (HGF). Left column, VEGF/DAPI; right column, HGF/DAPI; A and B, pocket portions; C and D, around a columnar member; E and F, center part.
[Fig. 16] Fig. 16 presents photographs showing the results of dual staining of the tissue construct for the stem cell marker SSEA3 and other markers. A, CD90/SSEA3/DAPI; B, CD105/SSEA3/DAPI; C, SSEA3/SSEA4/DAPI; D, VEGF/SSEA3/DAPI.
[Fig. 17] Fig. 17 shows the results of flow cytometric analysis.
[Fig. 18] Fig. 18 shows an increased proportion of CD90-positive cells as shown by flow cytometric analysis of cell population before cell separation and after cell separation.
[Fig. 19] Fig. 19 shows an increased proportion of SSEA3-positive cells as shown by flow cytometric analysis of cell population before cell separation and after cell separation.
[Fig. 20] Fig. 20 presents photographs of cell morphology of cell population before cell separation (control; the left column), CD90-positive cell population (the center column), and SSEA3-positive cell population (the right column) on day 7 (upper photographs) and day 14 (lower photographs) after the initiation of culture thereof. When cells separated by the MACS method were cultured, fibroblast-like cells adhered to the plate and proliferated.
[Fig. 21] Fig. 21 presents photographs and diagrams showing the outline of the production test of the tissue construct using a stent.
[Fig. 22] Fig. 22 presents a cross-sectional photograph of the tissue construct formed in the device for producing a tissue construct.
[Fig. 23] Fig. 23 shows the association between the number of somatic stem cells and the duration of placing, by the observation images.
[Fig. 24] Fig. 24 presents photographs showing HE-stained images of sections of the tissue construct formed in the device for producing a tissue construct of Fig. 4A.
[Fig. 25] Fig. 25 presents photographs showing HE-stained images of sections of the tissue construct formed in the device for producing a tissue construct of Fig. 4B.
[Fig. 26] Fig. 26 presents photographs showing HE-stained images of sections of the tissue construct formed in the device for producing a tissue construct of Fig. 4C.
[Fig. 27] Fig. 27 presents photographs showing HE-stained images of sections of the tissue construct formed in the device for producing a tissue construct of Fig. 5A.
[Fig. 28] Fig. 28 presents photographs showing HE-stained images of sections of the tissue construct formed in the device for producing a tissue construct of Fig. 5B.
[Fig. 29] Fig. 29 presents an enlarged image of a photograph of an HE-stained section having a cross section in the axial direction Ax of the tissue construct formed in the device for producing a tissue construct of Fig. 5B.
[Fig. 30] Fig. 30 presents photographs showing the appearance of the tissue construct formed in the device for producing a tissue construct of Fig. 6C and the positions of cross sections of two types of sections thereof.
[Fig. 31] Fig. 31 presents photographs showing HE-stained images of different sections having different cross-sections of the tissue construct formed in the device for producing a tissue construct of Fig. 6C.

### Description of Embodiments of the Invention

Hereafter, the present invention is described in detail.

The present invention relates to a device for producing a tissue construct capable of efficiently accumulating somatic stem cells from body tissue, and a tissue construct that is rich in somatic stem cells and can be prepared using such device.

For example, the present invention relates to a device for producing a tissue construct comprising:
a hollow portion; and
a frame body that forms the hollow portion,
   wherein the frame body has an opening, preferably, an opening that communicates from the hollow portion to an external space of the device for producing a tissue construct, and
   the tissue construct comprises a core portion having a recess and composed of fibrous connective tissue, and loose fibrous somatic stem cell-accumulated tissue comprising type III collagen and somatic stem cells which is formed in the recess. The present invention also relates to a tissue construct produced with the use of such device for producing a tissue construct.

According to the present invention, the placement of the device for producing a tissue construct as described above in an environment comprising body tissue materials (e.g., in a living body/*in vivo)* enables cells to be the accumulated in the device for producing a tissue construct, and formation of a tissue construct comprising somatic stem cells accumulated therein to be induced in the device for producing a tissue construct.

Through the opening of the device for producing a tissue construct of the present invention placed in an environment comprising body tissue materials, cells and the like from body tissue materials enter a hollow portion (which is open to the external space of the device) of the device, and fibrous connective tissue is formed in the hollow portion. A recess of the fibrous connective tissue recessed from the opening toward the hollow portion of the device is formed directly below the opening of the device for producing a tissue construct, and the core portion having the recess and composed of fibrous connective tissue is formed from the surface of the device facing the hollow portion of the device toward the inside of the hollow portion of the device, and many somatic stem cells are accumulated in the recess, thereby forming the somatic stem cell-accumulated tissue. The placement of the device for producing a tissue construct in an environment comprising body tissue materials for a duration enough to form tissue (comprising fibrous connective tissue and somatic stem cell-accumulated tissue in the recess(es) thereof) to fill the hollow portion in the device for producing a tissue construct results in formulation of a tissue construct in the device. It is possible to easily and efficiently collect somatic stem cells from the somatic stem cell-accumulated tissue in the resulting tissue construct. The present invention is based on the findings by the present inventors as described above.

More specifically, the device for producing a tissue construct of the present invention comprises a frame body that forms a hollow portion of the device (e.g., composed of the frame body), and the frame body has an opening that communicates from the hollow portion to the external space of the device for producing a tissue construct. The device for producing a tissue construct is, preferably, configured to allow fibrous connective tissue to be formed to stretch from the surface of the frame body toward the inside of the hollow portion, thereby causing the core portion having a recess recessed from the opening toward the hollow portion to be formed, and to allow loose fibrous tissue where somatic stem cells are accumulated to be formed in the recess, when the device for producing a tissue construct is placed in an environment comprising body tissue materials (e.g., in a living body/*in vivo*)*.* When a tissue construct is formed in the device for producing a tissue construct to fill the hollow portion formed by the frame body, the frame body defines the shape of a tissue construct (e.g., the shape of the whole tissue construct, the shape of the core portion, and the positions and the depths of the somatic stem cell-accumulated tissue/the recesses) formed in the hollow portion.

In an embodiment, the frame body may comprise a columnar member and a supporting member, and, for example, it may be composed of, a columnar member and a supporting member. The supporting member fixes the columnar member to maintain the shape of the frame body, thereby maintaining the shape of the hollow portion. A plurality of the columnar members, for example, may be fixed to the supporting member at the both ends of the columnar members. A plurality of the columnar members may further be fixed to a supporting member at the intermediate parts of the columnar members. The frame body preferably comprises a plurality of the columnar members. For example, the frame body may comprise 3 to 50, 4 to 40, 4 to 30, 5 to 30, 3 to 20, 3 to 15, 3 to 10, 4 to 15, or 5 to 15 columnar members, although the number of columnar members is not limited thereto. The columnar member may have any shape such as, circular cylinder, elliptic column, prism, frustum, twist, or screw. The supporting member may be in any shape of, for example, a ring, disk, oval ring, oval disk, rectangular frame, or plate (e.g., a polygonal, trapezoidal, or atypical plate). The frame body may comprise 1, 2, 3, 4, or more supporting members. The frame body may comprise 2 or more structures tiered in the extending direction of the columnar members, each of the structures comprises a plurality of the columnar members fixed to a supporting member at the both ends of the columnar members and optionally at the intermediate parts (e.g., at 1, or 2 or more sites on the intermediate parts) of the columnar members. In such a case, for example, 3 to 20, 3 to 15, 3 to 10, 4 to 15, or 5 to 15 columnar members may be fixed to one supporting member, although the number of the columnar members to be fixed thereto is not limited thereto.

In an embodiment, in the frame body, a plurality of the columnar members may be fixed to a supporting member at the both ends of the columnar members and optionally at the intermediate parts of the columnar members and may be arranged in a circumferential direction on a hypothetical cylindrical plane (e.g., on a hypothetical circular cylindrical, oval cylindrical, or square cylindrical plane) sandwiched between the supporting members. In such a case, the supporting member may be in the shape of a ring, disk, oval ring, oval disk, rectangular frame, or plate (e.g., a polygonal, trapezoidal, or atypical plate), although the shape is not limited thereto. The plurality of the columnar members may be arranged at equal or different intervals on a hypothetical cylindrical plane sandwiched between the supporting members. The frame body may comprise 2 or more structures tiered in the extending direction of the columnar members, each of the structures comprises a plurality of the columnar members that are fixed to a supporting member at the both ends of the columnar members and optionally at the intermediate parts of the columnar members and arranged in the circumferential direction on a hypothetical cylindrical plane sandwiched between the supporting members. In such a case, for example, 3 to 20, 3 to 15, 3 to 10, 4 to 15, or 5 to 15 columnar members may be fixed to one supporting member, although the number of the columnar members to be fixed is not limited thereto.

In another embodiment, the frame body may comprise a plurality of the columnar members. For example, the frame body may be composed of a plurality of the columnar members, and the columnar members may be fixed to each other to maintain the shape of the frame body.

A columnar member being a component of the frame body may have a sectional width of at least 0.5 mm, or 0.5 mm to 5 mm, for example, 0.7 mm to 3 mm, or 0.7 mm to 1.5 mm, although the sectional width is not limited thereto.

A supporting member being a component of the frame body may have a sectional width of 5 mm to 30 mm, for example, 5 mm to 25 mm or 6 mm to 20 mm, although the sectional width is not limited thereto.

In another embodiment, the frame body may comprise a mesh or coiled linear member, and for example, may be composed of a mesh linear member. The linear member may have a sectional width of 0.5 mm to 5 mm, for example, 0.7 mm to 3 mm or 0.7 mm to 1.5 mm, although the sectional width is not limited thereto. The frame body may comprise a linear member and a supporting member, and, for example, the frame body may be composed of a linear member and a supporting member. Such supporting member fixes the linear member to maintain the shape of the frame body.

In the present invention, the term "sectional width" shall be defined as the longest distance between 2 parallel tangential lines that sandwich a section (in general, a transverse cross section) of a member or region of interest from the both sides, unless otherwise specified. The form of the cross section is not limited to a circular or elliptic form, and it may be, for example, a polygonal, trapezoidal, or atypical form.

The length in the longitudinal direction of the device for producing a tissue construct, the frame body, and the columnar member being a component of the device and the frame body may be 5 mm to 100 mm, for example, 15 mm to 90 mm, 20 mm to 80 mm, 15 mm to 70 mm, or 20 mm to 60 mm, although the length is not limited thereto.

The device for producing a tissue construct, the frame body, and the components thereof such as the columnar member, the supporting member, and the linear member, preferably comprise or are made of a biocompatible material. Examples of the biocompatible material include, but are not limited to, resin materials, such as acrylic resin or silicone resin, ceramics, metal materials such as stainless steel, cobalt-chromium alloy, titanium, titanium alloy materials (e.g., nickel-titanium alloy), platinum, and gold. The biocompatible material used in this context of the present invention preferably has rigidity to the extent that the biocompatible material would not be deformed in a living body at least within a period for production of the tissue construct.

The frame body may have 1, 2, 3, or 4 or more (e.g., 4, 5, 6, 7, 8, 9, or 10 or more) openings that communicates from the hollow portion of the device to an external space of the device for producing a tissue construct, in which a recess of the fibrous connective tissue being formed directly below the opening. For example, the number of the openings may be 1 to 50, 3 to 50, 4 to 40, 4 to 30, 5 to 30, 3 to 20, 3 to 15, 3 to 10, 4 to 15, or 5 to 15. The opening may be preferably formed by a columnar member and a supporting member, or by a plurality of columnar members. Alternatively, the opening may be formed by a linear member, or by a linear member and a supporting member.

The supporting member itself of the frame body may have an opening that communicates from the hollow portion of the device to an external space of the device. For example, when the supporting member is in the shape of a ring, oval ring, rectangular frame, or the like, the supporting member may have the opening.

The opening of the frame body has an opening width that is wide enough to form the fibrous connective tissue/core portion, the recess of the fibrous connective tissue/core portion, and somatic stem cell-accumulated tissue in the recess. In the present invention, the term "opening width" of the opening of the frame body is defined as an opening width in the transverse direction of the opening. The opening of the frame body preferably has an opening width of at least 2.5 mm, for example, an opening width of 3.0 mm or larger. In an embodiment, the opening may have an opening width of 2.5 mm to 28 mm; 2.5 mm to 25 mm; 2.5 mm to 20 mm; or 3.0 mm to 10 mm. The opening width of the opening of the frame body is also referred to as the "opening width W" herein.

In the present invention, the terms "longitudinal (direction)" and "transverse (direction)" are used for any form or an object with any form. For example, such terms are used for various shapes or objects such as, in addition to a rectangle or cuboid, other polygons, circle, oval, circular cylinder, prism, cone, polygonal pyramid, frustum, and so on, and frame bodies having such shapes. In some shapes or objects, such as a circle, square, or cube, the length in the "longitudinal direction" may be the same as the length in the "transverse direction."

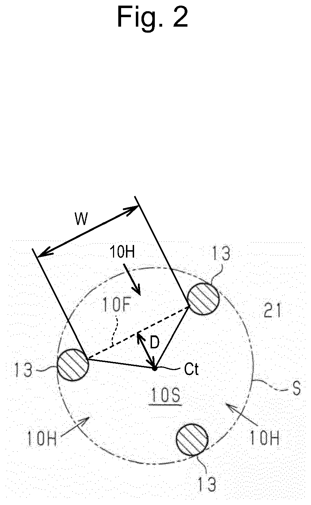

The opening width of the opening of the frame body can be determined at the opening edge that is defined by points positioned on the component(s) (e.g., columnar member(s), supporting member(s), and/or linear member(s)) forming the opening, and in the same plane where the opening has the smallest opening area (see, for example, Fig. 2, 10F). When the frame body is composed of a plurality of columnar members and a supporting member or of a plurality of columnar members or linear members, the opening width of the opening can be determined as the shortest distance between the columnar members or linear members adjacent to each other on a transverse cross section plane including the opening of the frame body. In this case, the transverse cross section plane of the frame body may be parallel to the supporting member. When columnar members or linear members adjacent to each other are arranged in parallel, the opening width of the opening can be determined as the single shortest distance between the columnar members or linear members.

Alternatively, or in addition to the above, the opening of the frame body preferably has an opening configuration (a shape and size) that enables a circle with a diameter of 2.5 mm or larger (e.g., a diameter of 3.0 mm or larger) to be inscribed in the opening. The opening of the frame body may have an opening configuration that enables a circle with a diameter of, for example, 2.5 mm to 28 mm, 2.5 mm to 25 mm, 2.5 mm to 20 mm, or 3.0 mm to 10 mm, to be inscribed in the opening. The opening of the frame body having such opening configuration is particularly suitable to form a recess of the fibrous connective tissue and somatic stem cell-accumulated tissue in the recess. The opening of the frame body having "an opening configuration that enables a circle to be inscribed in the opening" used herein means an opening where a circle having a diameter of a given size can fit inside the opening configuration (e.g., a rectangular or polygonal configuration) of the opening, in contact with the opening at one or more points. For example, an opening with a rectangular configuration (longer side: 20 mm; shorter side: 2.5 mm) enables a circle with a diameter of up to 2.5 mm to be inscribed in the opening. In the present invention, an opening configuration of the opening of the frame body can be defined at positions on the opening where the opening has the smallest opening area. For example, such configuration can be defined on any plane (e.g., a plane) including the opening edge.

In an embodiment, the opening of the frame body may have an opening edge of any configuration, for example, having a polygonal, rectangular, trapezoidal, round, circular, or elliptic shape.

In at least one opening of the frame body of the device for producing a tissue construct according to the present invention, opposite sides of the opening edge may provide opening widths varying in the extending direction of the sides. When the device for producing a tissue construct has such configuration, a single opening can have varying opening widths.

The hollow portion formed by the frame body preferably has a sufficient depth to form the core portion. In the frame body, the depth of the hollow portion from the opening may be at least 2 mm. The depth of the hollow portion from the opening may be, for example, 2 mm to 30 mm, 2 mm to 26 mm, or 2 mm to 12 mm. The depth of the hollow portion from the opening in the frame body (referred to as the "depth D" herein) is determined, on a transverse cross section plane of the frame body, as the distance from the midpoint of the straight line connecting points located on the opening edge of a single opening, to the deepest point of the hollow portion (Fig. 2). When a plurality of the columnar members are fixed at the both ends and optionally at the intermediate parts thereof to the supporting member, and arranged in a circumferential direction on a hypothetical cylindrical plane sandwiched between the supporting members (e.g., so as to be inscribed in or circumscribed to the hypothetical cylindrical plane; preferably, to surround the hypothetical cylindrical plane) in the frame body, the center point of the transverse cross section of the cylindrical plane can be determined to be the deepest point of the hollow portion. The center point of the transverse cross section in the transverse direction may be a point at the same distance from each columnar member arranged in the circumferential direction on the hypothetical cylindrical plane. The center point of the transverse cross section may be the center of a circular section of the hypothetical cylindrical plane. The device for producing a tissue construct according to the present invention may comprise at least one opening with the depth D of 2 mm or more (preferably, when the depth D of the opening is constant) or with the maximum depth D of 2 mm or more (when the depth D of the opening is not constant).

Concerning the device for producing a tissue construct of the present invention, at least one the openings of the frame body is required to be configured as described above, and preferably all the openings of the frame body be configured as described above.

In the frame body of the device for producing a tissue construct according to the present invention, a plurality of the openings may be positioned adjacent to each other while sandwiching a columnar member. A plurality of the openings may be arranged along the circumferential direction of the frame body. Alternatively, a plurality of the openings may be arranged along the axial direction of the frame body. Alternatively, a plurality of the openings may be arranged along the circumferential direction and along the axial direction of the frame body. Such opening configuration is capable of increasing the density of the recesses to be formed on the outer part of the tissue construct, which are intended to accumulate somatic stem cells therein. In a preferred embodiment, accordingly, the opening configuration also enables the efficiency of somatic stem cell accumulation to be increased, and, in turn, the number of somatic stem cells collected with the device to be increased. When such device for producing a tissue construct is used, the core portion of the resulting tissue construct may comprise a plurality of the recesses separated by recess peripheries. In that case, each recess is adjacent to another recess across at least one recess edge.

It is preferred that the device for producing a tissue construct according to the present invention do not comprise other members such as rod-like members in the hollow portion formed by the frame body. It is preferred that the device would be configured not to be placed in an environment comprising body tissue materials in a state where other members such as rod-like members are set in the hollow portion of the device.

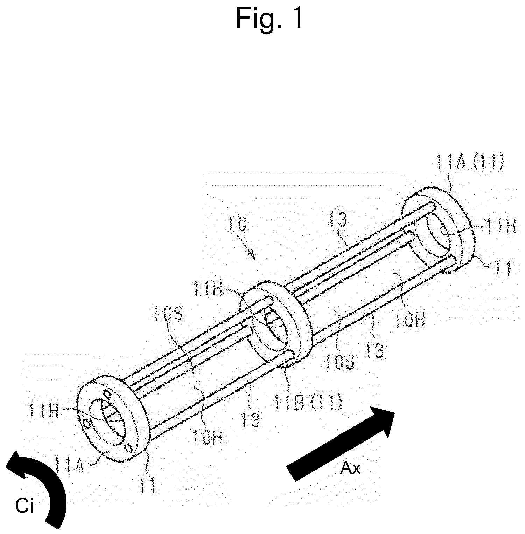

In an embodiment, the device for producing a tissue construct according to the present invention is one as illustrated in Fig. 1. As shown in Fig. 1, the device for producing a tissue construct 10 (hereafter, the device for producing a tissue construct according to the present invention may also be referred to as device 10) comprises three ring-shaped supporting members 11. Each supporting member 11 may comprise or may be made of biocompatible resin such as, but not limited to, acrylic resin or silicone resin. The device comprises the three supporting members 11 which are composed of two first supporting members 11A, and one second supporting member 11B located between the two first supporting members 11A; and the three supporting members 11 are aligned along the single axial direction Ax. The first supporting member 11A and the second supporting member 11B are located at the both ends of the same hypothetical circular cylindrical plane S. The two first supporting members 11A each have a through-hole 11H that communicates from the outside of the device to the inside of the device in the axial direction Ax. The second supporting member 11B has a through-hole 11H that communicates from the inside of one circular cylindrical plane S to the inside of another circular cylindrical plane S in the axial direction Ax. When the device 10 is implanted and placed in an environment comprising body tissue materials, such as in body tissue in a living body, cells and the like including fibroblasts can flow into the device toward the hollow portion thereof from the through-holes 11H.

One first supporting member 11A and one second supporting member 11B fix 3 circular cylindrical columnar members 13. The 3 columnar members 13 are arranged at equal intervals in the circumferential direction Ci on each circular cylindrical plane S, and 3 openings 10H are defined and formed by the 3 columnar members 13 together with the first supporting member 11A and the second supporting member 11B. The columnar members 13, the 2 first supporting members 11A, and the second supporting member 11B constitute the frame body of the device 10. Before the device 10 is implanted in an environment comprising body tissue materials, such as in body tissue in a living body, a space surrounded by the columnar members 13, the first supporting member 11A, and the second supporting member 11B is the hollow portion 10S, and each opening 10H communicates from the hollow portion 10S of the device to the external space of the device. When the device 10 is implanted in an environment comprising body tissue materials, such as in body tissue in a living body, cells and the like including fibroblasts can flow into the device from the body tissue toward the hollow portion 10S of the device through the through-holes 11H.

Fig. 2 schematically shows a transverse cross section of the device as illustrated in Fig. 1 including the opening. As seen in Fig. 2, the length of a straight line that connects points on the opening edge 10F on the cross section, which can be the shortest distance between the columnar members 13 adjacent to each other in the circumferential direction on the circular cylindrical plane S, corresponds to the opening width W. When the adjacent columnar members 13 are arranged in parallel, the opening width W is constant along the axial direction Ax. When the adjacent columnar members 13 are not arranged in parallel, the opening width W varies between positions along the axial direction Ax. The opening of the device 10 may be an opening configuration having the opening width W of 2.5 mm or larger, for example, 2.5 mm to 28 mm, 2.5 mm to 25 mm, 2.5 mm to 20.0 mm, or 3.0 mm to 10 mm. In terms of further enhancement of the efficiency of somatic stem cell accumulation, the opening width W is preferably at least 2.5 mm throughout the axial direction Ax of the device 10. Alternatively, or in addition to the above, the opening of the device 10 preferably has an opening configuration that enables a circle with a diameter of 2.5 mm to 28 mm, 2.5 mm to 25 mm, 2.5 mm to 20 mm, or 3.0 mm to 10 mm to be inscribed in the opening.

The distance between the center (center point) Ct on the cylindrical plane S on the cross section illustrated in Fig. 2 and the midpoint of the line that connects points on the opening edge 10F corresponds to the depth D of the hollow portion 10S from the opening 10H. The device 10 may have the opening 10H with the depth D or the maximum depth which is at least 2 mm, for example, 2 mm to 30 mm, 2 mm to 26 mm, or 2 mm to 12 mm.

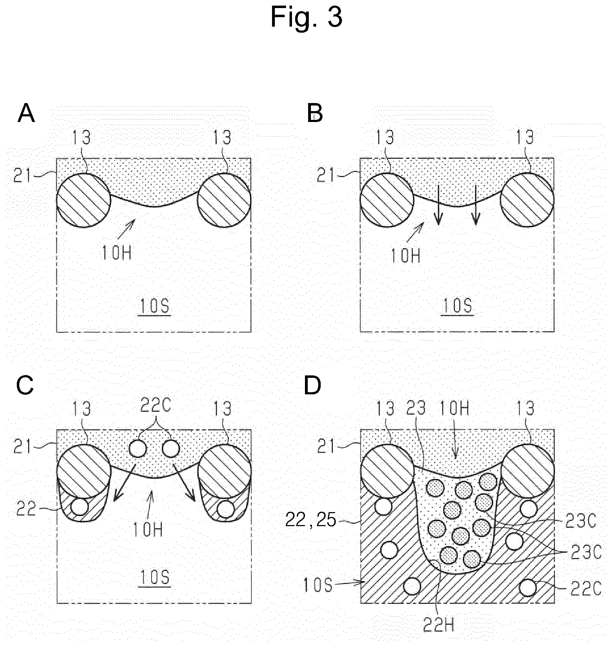

In the device 10 for producing a tissue construct implanted in an environment comprising body tissue materials, such as in body tissue in a living body, tissue formation is considered to occur as summarized in Fig. 3, although the present invention is not constrained by the theory. As shown in Fig. 3A, when the device 10 is implanted in an environment comprising body tissue materials, such as in body tissue in a living body, the body tissue 21 is positioned outside the hollow portion 10S. Subsequently, the body tissue-derived humoral components infiltrate into the hollow portion 10S through the opening 10H and the through-hole 11H, as shown in Fig. 3B. After an adequate period of time, for example, 3 to 6 hours following the implantation of the device 10 in body tissue, the hollow portion 10S of the device 10 is filled with the body tissue-derived humoral components. As shown in Fig. 3C, when the hollow portion 10S of the device 10 is filled with the humoral components, fibroblasts 22C in body tissue infiltrate into the hollow portion 10S through the opening 10H and the through-hole 11H. The fibroblasts 22C that had infiltrated into the hollow portion 10S begin to form a fibrous connective tissue 22 from the surface of the columnar members 13 toward the inside of the hollow portion 10S.

As shown in Fig. 3D, the fibrous connective tissue that has begun to be formed on the surface of the columnar member 13 facing the hollow portion 10S grows to stretch toward the inside of the hollow portion 10S. The fibrous connective tissues 22 stretched from the surfaces of the columnar members 13 are joined to each other within the hollow portion 10S, thereby causing the recess 22H that is recessed from the opening 10H toward the hollow portion 10S to be formed on the surface of the fibrous connective tissue 22. Also from the surface of the supporting member 11, which is a component element of the device 10 for producing a tissue construct, for example, the first supporting member 11A and the second supporting member 11B of the device illustrated in Fig. 2, toward the inside of the hollow portion 10S, the fibrous connective tissue 22 is formed, grown, and joined to the fibrous connective tissue 22 stretched from the surface of the columnar member 13. The fibrous connective tissues 22 formed to stretch from the surfaces of the columnar member 13 and the supporting member 11 (e.g., the first supporting members 11A and the second supporting member 11B of the device as illustrated in Fig. 2) are joined to each other to form a core portion 25. Therefore, the core portion 25 composed of the fibrous connective tissue 22 has a recess on its outer surface. As the formation of the fibrous connective tissue 22 and the core portion 25 having the recess advances, the somatic stem cells 23C included in body tissue migrate to the recess 22H through the opening 10H. The device 10 for producing a tissue construct implanted in an environment comprising body tissue materials, such as in body tissue in a living body, is placed for an adequate period of time, thereby resulting in the formation of the somatic stem cell-accumulated tissue (comprising the somatic stem cells 23C) formed in the recess, and such somatic stem cell-accumulated tissue is also referred to as a "pocket portion."

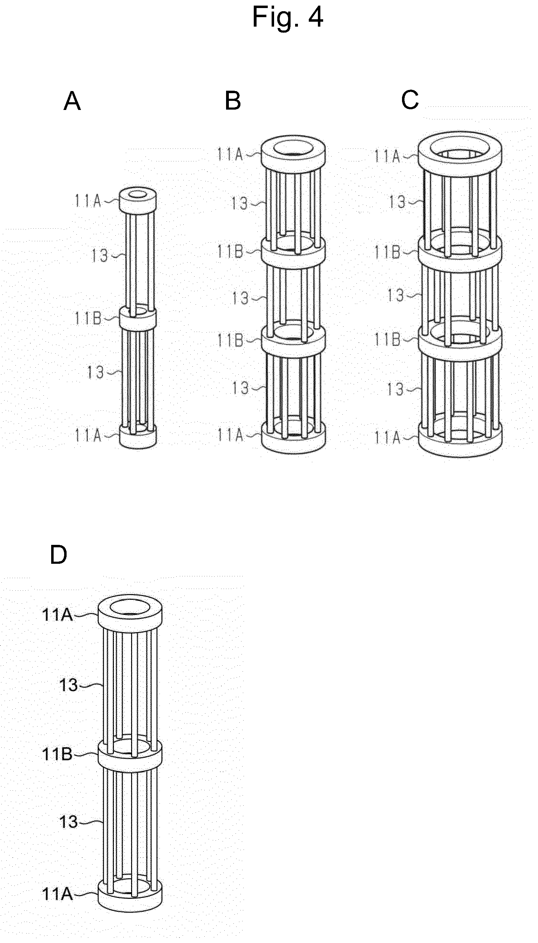

In another embodiment of the device 10 for producing a tissue construct, as shown in Fig. 4A, the device 10 may comprise columnar members 13, two ring-shaped first supporting members 11A with the outside diameter (i.e., the diameter of the outer circle) of 6 mm, and one ring-shaped second supporting member 11B with the outside diameter of 6 mm located between the two ring-shaped first supporting members 11A. Between one first supporting member 11A and the second supporting member 11B in the upper part of the device, three columnar members 13 with a diameter of 1 mm (length: 25 mm) are fixed so as to have the opening width W of 3.0 mm and the depth D of 2 mm. Between the first supporting member 11A and the second supporting member 11B in the lower part of the device, four columnar members 13 with a diameter of 1 mm (length: 25 mm) are fixed so as to have the opening width W of 2.5 mm and the depth D of 2.2 mm. In the device 10 as illustrated in Fig. 4A, each of the openings formed by the first supporting member 11A, the second supporting member 11B, and the columnar members 13 in the upper part has an opening configuration that enables a circle with a diameter of up to 3.0 mm (equal to the opening width W) to be inscribed in the opening, and each of the openings formed by the first supporting member 11A, the second supporting member 11B, and the columnar members 13 in the lower part has an opening configuration that enables a circle with a diameter of up to 2.5 mm (equal to the opening width W) to be inscribed in the opening.

In a further embodiment of the device for producing a tissue construct, as shown in Fig. 4B, the device 10 may comprise columnar members 13, two ring-shaped first supporting members 11A with the outside diameter of 11 mm, and two ring-shaped second supporting members 11B with the outside diameter of 11 mm located between the two ring-shaped first supporting members 11A. Between one first supporting member 11A and the second supporting member 11B in the upper part of the device, six columnar members 13 with a diameter of 1 mm (length: 20 mm) are fixed so as to have the opening width W of 4.0 mm and the depth D of 4.6 mm. Between the first supporting member 11A and the second supporting member 11B in the lower part of the device, eight columnar members 13 with a diameter of 1 mm (length: 20 mm) are supported so as to have the opening width W of 3.0 mm and the depth D of 4.8 mm. Between the two second supporting members 11B in the middle part of the device, four columnar members 13 with a diameter of 1 mm (length: 20 mm) are fixed so as to have the opening width W of 6.0 mm and the depth D of 4.0 mm. In the device 10 as illustrated in Fig. 4B, each of the openings formed by the first supporting member 11A, the second supporting member 11B, and the columnar members 13 in the upper part has an opening configuration that enables a circle with a diameter of up to 4.0 mm (equal to the opening width W) to be inscribed in the opening; each of the openings formed by the first supporting member 11A, the second supporting member 11B, and the columnar members 13 in the lower part has an opening configuration that enables a circle with a diameter of up to 3.0 mm (equal to the opening width W) to be inscribed in the opening; and each of openings formed by the two second supporting members 11B and the columnar members 13 in the middle part has an opening configuration that enables a circle with a diameter of up to 6.0 mm (equal to the opening width W) to be inscribed in the opening.

In a further embodiment of the device for producing a tissue construct, as shown in Fig. 4C, the device 10 may comprise columnar members 13, two ring-shaped first supporting members 11A with the outside diameter of 16 mm, and two ring-shaped second supporting members 11B with the outside diameter of 16 mm located between the two ring-shaped first supporting members 11A. Between one first supporting member 11A and the second supporting member 11B in the upper part of the device, eight columnar members 13 with a diameter of 1 mm (length: 20 mm) are fixed so as to have the opening width W of 5.0 mm and the depth D of 7.1 mm. Between the first supporting member 11A and the second supporting member 11B in the lower part of the device, ten columnar members 13 with a diameter of 1 mm (length: 20 mm) are fixed so as to have the opening width W of 3.5 mm and the depth D of 7.3 mm. Between the two second supporting members 11B in the middle part of the device, six columnar members 13 with a diameter of 1 mm (length: 20 mm) are fixed so as to have the opening width W of 6.5 mm and the depth D of 6.8 mm. In the device 10 as illustrated in Fig. 4C, each of the openings formed by the first supporting member 11A, the second supporting member 11B, and the columnar members 13 in the upper part has an opening configuration that enables a circle with a diameter of up to 5.0 mm (equal to the opening width W) to be inscribed in the opening; and each of the openings formed by the first supporting member 11A, the second supporting member 11B, and the columnar members 13 in the lower part has an opening configuration that enables a circle with a diameter of up to 3.5 mm (equal to the opening width W) to be inscribed in the opening; and each of the openings formed by the two second supporting members 11B and the columnar members 13 in the middle part has an opening configuration that enables a circle with a diameter of up to 6.5 mm (equal to the opening width W) to be inscribed in the opening.

In a further embodiment of the device 10 for producing a tissue construct, as shown in Fig. 4D, the device 10 may comprise columnar members 13, two ring-shaped first supporting members 11A with the outside diameter of 11 mm, and one ring-shaped second supporting member 11B with the outside diameter of 10 mm located between the two ring-shaped first supporting members 11A. Between the two first supporting members 11A, six columnar members 13 with a diameter of 1 mm (length: 60 mm) are fixed so as to have the opening width W of 4.0 mm and the depth D of 4.6 mm, and these columnar members 13 are further fixed by the second supporting member 11B approximately at the center of the columnar members 13 in the longitudinal direction. In the device 10 as illustrated in Fig. 4D, each of the openings formed by the first supporting member 11A, the second supporting member 11B, and the columnar members 13 has an opening configuration that enables a circle with a diameter of up to 4.0 mm to be inscribed in the opening.

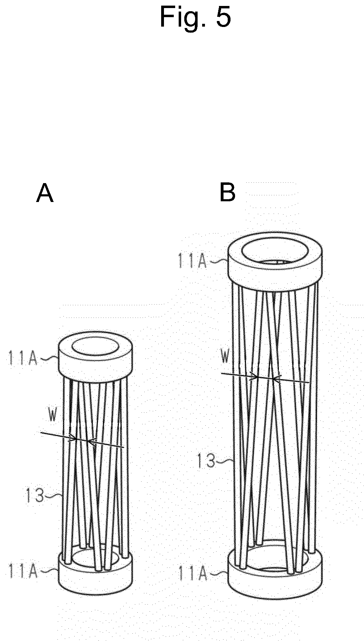

In a further embodiment of the device for producing a tissue construct, as shown in Fig. 5A, the device 10 may comprise columnar members 13 and two ring-shaped first supporting members 11A with the outside diameter of 11 mm. Between the two first supporting members 11A, eight columnar members 13 with a diameter of 1 mm are fixed so that the opening width W would continuously vary from 1.0 mm to 5.0 nm and the depth D would continuously vary from 5.0 mm to 4.3 mm from one first supporting member 11A toward the other first supporting member 11A. The opening width W of this embodiment is measured on a transverse cross section parallel to the supporting member 11A of the device 10. The length (the size in the longitudinal direction) of the device 10 as illustrated in Fig. 5A is 40 mm.

In a further embodiment of the device for producing a tissue construct, as shown in Fig. 5B, the device 10 may comprise columnar members 13 and two ring-shaped first supporting members 11A with the outside diameter of 16 mm. Between the two first supporting members 11A, eight columnar members 13 with a diameter of 1 mm are fixed so that the opening width W would continuously vary from 1.0 mm to 8.0 nm and the depth D would continuously vary from 7.5 mm to 6.3 mm from one first supporting member 11A toward the other first supporting member 11A. The opening width W of this embodiment is measured on a transverse cross section parallel to the supporting member 11A of the device 10. The length (the size in the longitudinal direction) of the device 10 as illustrated in Fig. 5B is 60 mm.

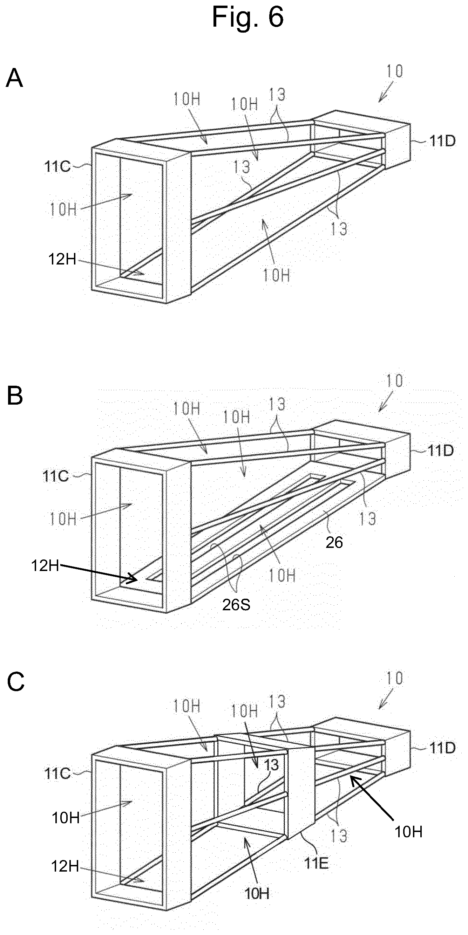

In a further embodiment of the device for producing a tissue construct, as shown in Fig. 6A, the device 10 may have a generally polyhedral shape, which is composed of a large rectangular frame 11C as an example of the supporting member, a small rectangular frame 11D as another example of the supporting member, and columnar members 13 (diameter: 1 mm). The large rectangular frame 11C has a size of 20 mm (vertical) x 15 mm (horizonal), which are both inner dimensions, and the thickness of the rectangular frame is 1 mm and the outer dimensions are 22 mm (vertical) x 17 mm (horizonal). The small rectangular frame 11D has a size of 3 mm (vertical) x 15 mm (horizonal), which are both inner dimensions, and the thickness of the rectangular frame is 1 mm and the outer dimensions are 5 mm (vertical) x 17 mm (horizonal). The device 10 as illustrated in Fig. 6A comprises four columnar members 13 that are fixed to the four vertexes of the large rectangular frame 11C and to the four vertexes of the small rectangular frame 11D. The device 10 as illustrated in Fig. 6A further comprises one columnar member 13 that connects a midpoint of one longer edge of the large rectangular frame 11C and a midpoint of one shorter edge of the small rectangular frame 11D.

The device 10 as illustrated in Fig. 6A comprises two openings 10H that are trapezoidal, and defined and formed by one longer edge of the large rectangular frame 11C, one shorter edge of the small rectangular frame 11D, and three columnar members 13 (the shortest length of the columnar members 13 connected perpendicularly to the large rectangular frame 11C and the small rectangular frame 11D: 55 mm) connecting thereto. The device 10 as illustrated in Fig. 6A comprises one opening 10H that is trapezoidal, and defined and formed by one longer edge of the large rectangular frame 11C, one shorter edge of the small rectangular frame 11D, and two columnar members 13 connected thereto. The device 10 as illustrated in Fig. 6A also comprises an opening 10H that is rectangular, and defined and formed by one shorter edge of the large rectangular frame 11C, one longer edge of the small rectangular frame 11D, and two columnar members 13 connected thereto. The device 10 as illustrated in Fig. 6A further comprises two pass-through openings 12H; one is defined and formed by two longer edges and two shorter edges of the large rectangular frame 11C and the other is defined and formed by two longer edges and two shorter edges of the small rectangular frame 11D. In the device 10 as illustrated in Fig. 6A, the opening width ranges from 1.0 mm to 20.0 mm, and the depth D ranges from 2.0 mm to 10.5 mm. The opening width W of this embodiment is measured on a transverse cross section parallel to the longer edges of the large rectangular frame 11C of the device 10.

As shown in Fig. 6B, the device 10 may has a generally polyhedral shape, which is composed of a large rectangular frame 11C as an example of the supporting member, a small rectangular frame 11D as another example of the supporting member, and columnar members 13 (diameter: 1 mm), as with the case of the device 10 as illustrated in Fig. 6A. The large rectangular frame 11C has a size of 20 mm (vertical) x 15 mm (horizonal), which are both inner dimensions, and the thickness of the rectangular frame is 1 mm, and the outer dimensions are 22 mm (vertical) x 17 mm (horizonal). The small rectangular frame 11D has a size of 3 mm (vertical) x 15 mm (horizonal), which are both inner dimensions, and the thickness of the rectangular frame is 1 mm, and the outer dimensions are 5 mm (vertical) x 17 mm (horizonal). The device 10 as illustrated in Fig. 6B comprises two columnar members 13 that are fixed to two vertexes of the large rectangular frame 11C and two vertexes of the small rectangular frame 11D. In the device 10 as illustrated in Fig. 6B, one shorter edge of the large rectangular frame 11C and one longer edge of the small rectangular frame 11D are connected with one plate-like body 26 having a slit 26S (width: 1 mm).

The device 10 as illustrated in Fig. 6B comprises an opening 10H that is trapezoidal, and defined and formed by one longer edge of the large rectangular frame 11C, one shorter edge of the small rectangular frame 11D, and two columnar members 13 connected thereto. The device 10 as illustrated in Fig. 6B comprises an opening 10H that is rectangular, and defined and formed by one shorter edge of the large rectangular frame 11C, one longer edge of the small rectangular frame 11D, and two columnar members 13 connected thereto. The device 10 as illustrated in Fig. 6B comprises two openings 10H that are trapezoidal, and defined and formed by one longer edge of the large rectangular frame 11C, one shorter edge of the small rectangular frame 11D, an edge of the plate-like body connecting the frames to each other, and columnar members 13. The device 10 as illustrated in Fig. 6B further comprises two pass-through openings 12H; one is defined and formed by two longer edges and two shorter edges of the large rectangular frame 11C and the other is defined and formed by two longer edges and two shorter edges of the small rectangular frame 11D. In the device 10 as illustrated in Fig. 6B, the opening width ranges from 1.0 mm to 20.0 mm, and the depth D ranges from 2.0 mm to 20.5 mm. The opening width W of this embodiment is measured on a transverse cross section parallel to the longer edges of the large rectangular frame 11C of the device 10.

As shown in Fig. 6C, the device 10 may have a generally polyhedral shape, which is composed of a large rectangular frame 11C as an example of the supporting member, a small rectangular frame 11D as another example of the supporting member, a medium rectangular frame 1 1E as another example of the supporting member, and columnar members 13 (diameter: 1 mm). The large rectangular frame 11C has a size of 20 mm (vertical) x 15 mm (horizonal), which are both inner dimensions, and the thickness of the rectangular frame is 1 mm and the outer dimensions are 22 mm (vertical) x 17 mm (horizonal). The medium rectangular frame 11E has a size of 15 mm (vertical) x 15 mm (horizonal), which are both inner dimensions, and the thickness of the rectangular frame is 1 mm and the outer dimensions are 17 mm (vertical) x 17 mm (horizonal). The small rectangular frame 11D has a size of 3 mm (vertical) x 15 mm (horizonal), which are both inner dimensions, and the thickness of the rectangular frame is 1 mm, and the outer dimensions are 5 mm (vertical) x 17 mm (horizonal). The device 10 as illustrated in Fig. 6C comprises four columnar members 13 that are fixed to the four vertexes of the large rectangular frame 11C and the four vertexes of the small rectangular frame 11D, and the four columnar members 13 are further fixed by the medium rectangular frame 11E. The device 10 as illustrated in Fig. 6C further comprises one columnar member 13 that connects a midpoint of one longer edge of the large rectangular frame 11C and a midpoint of one shorter edge of the small rectangular frame 11D and is further fixed by the medium rectangular frame 11E at the intermediate part thereof.

The device 10 as illustrated in Fig. 6C comprises an opening 10H that is rectangular, and defined and formed by one shorter edge of the large rectangular frame 11C, one edge of the medium rectangular frame 11E, and two columnar members 13 connected thereto. The device 10 as illustrated in Fig. 6C comprises an opening 10H that is rectangular, and defined and formed by one edge of the medium rectangular frame 11E, one longer edge of the small rectangular frame 11D, and two columnar members 13 connected thereto. Also, the device 10 as illustrated in Fig. 6C comprises two openings 10H that are trapezoidal, and defined and formed by one longer edge of the large rectangular frame 11C, one edge of the medium rectangular frame 11E, and three columnar members 13 connected thereto. The device 10 as illustrated in Fig. 6C comprises two openings 10H that are trapezoidal, and defined and formed by one edge of the medium rectangular frame 11E, one shorter edge of the small rectangular frame 11D, and 3 columnar members 13 connected thereto. The device 10 as illustrated in Fig. 6C comprises an opening 10H that is rectangular, and defined and formed by one longer edge of the large rectangular frame 11C, one edge of the medium rectangular frame 11E, and two columnar members 13 connected thereto. The device 10 as illustrated in Fig. 6C comprises an opening 10H that is rectangular, and defined and formed by one edge of the medium rectangular frame 11E, one shorter edge of the small rectangular frame 11D, and two columnar members 13 connected thereto. The device 10 as illustrated in Fig. 6C further comprises two pass-through openings 12H; one is defined and formed by two longer edges and two shorter edges of the large rectangular frame 11C and the other is defined and formed by two longer edges and two shorter edges of the small rectangular frame 11D. In the device 10 as illustrated in Fig. 6C, the opening width ranges from 1.0 mm to 20.0 mm, and the depth D ranges from 2.0 mm to 10.5 mm. The opening width W of this embodiment is measured on a transverse cross section parallel to the longer edges of the large rectangular frame 11C of the device 10.

The positions of the openings 10H are not limited to positions aligned in the circumferential direction and/or the axial direction Ax of the device 10, and they may be helically and periodically positioned in the longitudinal direction of the device 10. The positions of the openings 10H are not necessarily configured to provide a repeat of single opening 10 in a given direction. For example, the positions of the openings 10H may be configured to form a group of openings composed of a plurality of openings 10H, and to provide a repeat of the group of openings in a given direction.

In a further embodiment of the device for producing a tissue construct, the shape of the device 10 or the frame body is not particularly limited, and it may be a rod-like shape, a generally polyhedral shape (e.g., a cube or cuboid), or a generally spherical or hemispherical shape. The shape of the device 10 or the frame body is not limited to a linear rod-like shape, and it may be a curved shape.

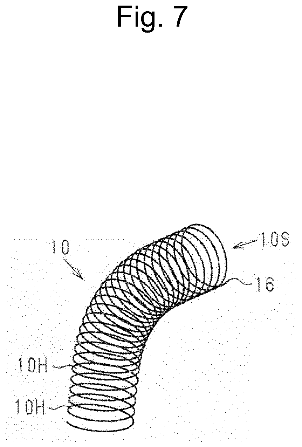

In a further embodiment of the device for producing a tissue construct, as shown in Fig. 7, the frame body being a component of the device 10 may be in the form of a coil, and may be in the form of one or more helixes with the opening edge 10F tiered in the circumferential direction of the coil. The opening width W of this embodiment can be measured as the shortest distance between adjacent circumferential parts of the linear member 16 in the form of a coiled coil. The device 10 preferably has an opening with an opening configuration having the opening width W of at least 2.5 mm, for example, 2.5 mm to 28 mm, 2.5 mm to 25 mm, 2.5 mm to 20 mm, or 3.0 mm to 10 mm. The shortest distance between the central axis of the device 10 in the form of a coil and the linear member 16 in the form of a coiled coil can be determined as the depth D. The device 10 may have a region in which the depth D is at least 2 mm, for example, 2 mm to 30 mm, 2 mm to 26 mm, or 2 mm to 12 mm.

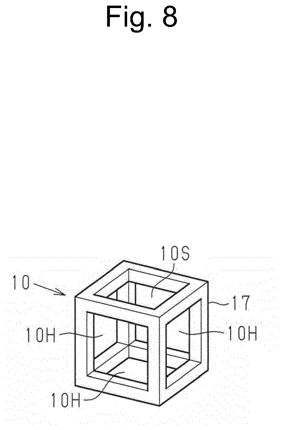

In a further embodiment of the device for producing a tissue construct, for example as shown in Fig. 8, the device 10 may be composed of a cubic or cuboid frame body having a hollow portion. A lateral face 17 of the cubic or cuboid frame body has the opening 10H, and the opening width W of this embodiment can be determined as the shortest distance between columnar members adjacent to each other being components of the lateral face 17. The device 10 or the frame body preferably has at least one opening with the opening width W of at least 2.5 mm, for example, 2.5 mm to 28 mm, 2.5 mm to 25 mm, 2.5 mm to 20 mm, or 3.0 mm to 10 mm. The shortest distance between the center point of the cubic or cuboid device 10 or the frame body and the lateral face of the device 10 can be determined as the depth D. The device 10 may have a region in which the depth D is at least 2 mm, for example, 2 mm to 30 mm, 2 mm to 26 mm, or 2 mm to 12 mm.

The frame body of the device 10 may have a configuration in which a plurality of the cubic or cuboid structures, for example, as illustrated in Fig. 8 are tiered on a single plane.

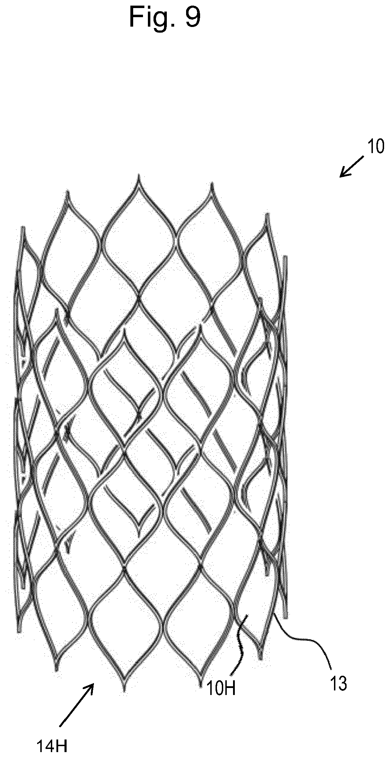

In a further embodiment of the device for producing a tissue construct, the device 10 may be in the form of a stent. The device 10 in the form of a stent as illustrated in Fig. 9 may have an expandable and contractible mesh cylindrical form in which the mesh provides the opening 10H and its both ends have end openings 14H for use in balloon insertion. In the present invention, for example, a device in the form of a stent as illustrated in Fig. 9 can be used as the device for producing a tissue construct. In a typical embodiment, a balloon catheter with a closed stent mounted thereon is inserted into an environment comprising body tissue materials, such as body tissue in a living body, the balloon is inflated subcutaneously by applying hydraulic pressure, to cause the stent to expand, and then hydraulic pressure is released, and the balloon is deflated and the catheter was withdrawn thereby enabling the stent to be easily implanted.

The present invention also relates to a method for producing a tissue construct comprising somatic stem cells accumulated therein, with the use of the device for producing a tissue construct according to the present invention. The method for producing a tissue construct comprising somatic stem cells accumulated therein with the use of the device for producing a tissue construct comprises placing the device for producing a tissue construct in an environment comprising body tissue materials, such as in a living body or in body tissue. More specifically, the method comprises positioning (e.g., implanting) the device for producing a tissue construct in an environment comprising body tissue materials, such as in a living body or in body tissue, and placing the device therein for a given period of time. The environment comprising body tissue materials, such as body tissue in a living body or isolated body tissue, may comprise fibroblasts. The body tissue may be ectodermal tissue, mesodermal tissue, or endodermal tissue. The body tissue in which the device for producing a tissue construct is to be placed may be a tissue in healthy conditions in which somatic stem cells are present, or a tissue such as a damaged or lost tissue in which somatic stem cells necessary for tissue repair are present. A preferred example of the body tissue comprising fibroblasts is subcutaneous tissue.

The device for producing a tissue construct can be implanted and placed in body tissue in a living body. Alternatively, the device for producing a tissue construct may be placed in an artificial environment that mimics the body tissue in a living body; specifically, an *in vitro* culture system comprising isolated body tissue from a living body, or an environment constructed outside a living body to mimic the body tissue in a living body. Examples of the artificial environment that mimics the body tissue in a living body include an artificial tissue or artificial organ prepared by three-dimensional culture of isolated cells or tissue.

When implanting and placing the device for producing a tissue construct in body tissue in a living body, a living body and/or body tissue is incised, under anesthesia as appropriate, to form an insertion opening. Subsequently, the device for producing a tissue construct is inserted into the insertion opening. After the device for producing a tissue construct is implanted, the incisional wound is sutured.

Body tissue in which the device for producing a tissue construct is positioned (e.g., implanted) can be from any animal from which somatic stem cells may be obtained. The device for producing a tissue construct can be positioned (e.g., implanted) and placed in a living body of any animal. Examples of the animal include, but are not limited to, mammalian animals, including primates such as human, monkey, and chimpanzee, and dog, cat, cow, pig, horse, goat, sheep, rat, and mouse; birds, fish, and amphibians. In an embodiment, such animal may be human, or non-human animals, such as non-human mammalian animals. In an embodiment, the device for producing a tissue construct may be placed in body tissue in a living body or isolated body tissue from a living body of an animal individual in which somatic stem cells collected from the tissue construct to be produced with the device for producing a tissue construct or cells differentiated from the collected somatic stem cells are to be implanted (i.e., an animal subject, such as a human or non-human animal) or an individual that is genetically closely related to the animal individual. This enables the implantation of autologous somatic stem cells or somatic stem cells derived from a closely related individual. In the present invention, a part in the living body in which the device for producing a tissue construct is to be implanted is not particularly limited. For example, the device may be implanted in abdomen, chest, shoulder, back, limbs, or abdominal cavity.

The device for producing a tissue construct positioned in an environment comprising body tissue materials is placed therein until a core portion having a recess and composed of fibrous connective tissue and a tissue comprising somatic stem cells accumulated in the recess are formed in the hollow portion of the device.

The period of time for which the device for producing a tissue construct is placed in an environment comprising body tissue materials, such as in body tissue in a living body or the isolated body tissue, is preferably enough to enable somatic stem cells to be sufficiently accumulated without causing excessive inflammation in body tissue. The period of time can be adequately determined depending on, for example, the site of implantation, the organism species of the living body, and/or the size of the device. The period of time for placement may be, in general, 5 days to 4 months, and preferably 1 week (7 days) to 3 months, for example, 1 week to 2 months, 1 week to 5 weeks, 9 days to 5 weeks, 9 days to 4 weeks, 2 weeks to 5 weeks, 2 weeks to 4 weeks, 2 weeks to 3 weeks, or 3 weeks to 5 weeks.

After the period of time for placement, the device for producing a tissue construct is removed from the environment comprising body tissue materials. In the removed apparatus for producing a tissue construct, a tissue construct comprising a core portion comprising fibrous connective tissue, with a recess that comprises tissue comprising somatic stem cells accumulated therein is formed.

When removing the device 10 from the living body, the site of implantation of the device for producing a tissue construct is incised, under anesthesia as appropriate. After the device for producing a tissue construct is removed from the site of incision, the incisional wound is preferably sutured.

By removing the device for producing a tissue construct from the environment comprising body tissue materials, such as from body tissue in a living body as described above, the tissue construct can be isolated. The tissue construct may be separated from the removed device for producing a tissue construct to collect the tissue construct. With the use of the device for producing a tissue construct, as described above, a tissue construct can be produced.

The present invention also provides the tissue construct according to the present invention that can be produced with the use of the above-mentioned device for producing a tissue construct. More specifically, the present invention relates to a tissue construct comprising a core portion having a recess and composed of fibrous connective tissue, and a loose fibrous tissue containing type III collagen and somatic stem cells which is formed in the recess (somatic stem cell-accumulated tissue).

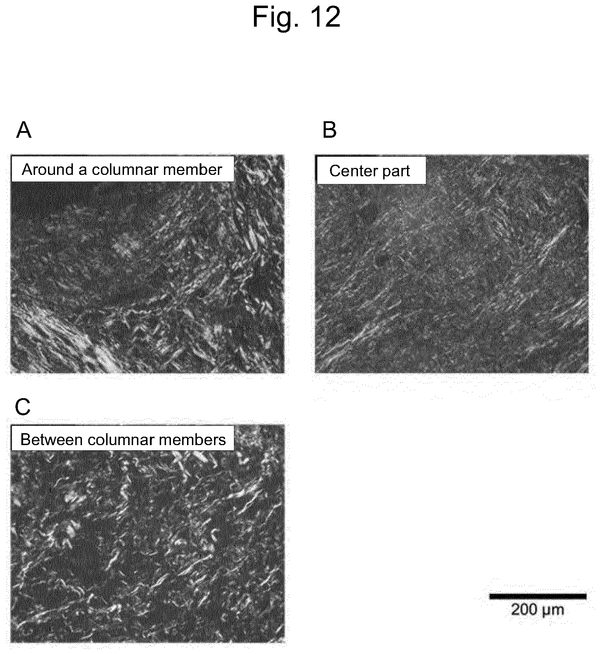

In the present invention, the fibrous connective tissue 22 composing the core portion 25 of the tissue construct is a rigid tissue with high fiber density that contains collagen fibers and fibroblasts as main components. In the present invention, the fibrous connective tissue composing the core portion can also be referred to as a "dense connective tissue," and it is different from adipose tissue or loose connective tissue. In the present invention, it is preferred that the fibrous connective tissue composing the core portion comprise type I collagen, as a main collagen component. In the present invention, on the other hand, the content of type III collagen in the core portion according to the present invention is typically low, and, in particular, the content of type III collagen in the center part of the core portion is only very low. In a preferred embodiment of the present invention, the proportion of type I collagen to the total collagen in the core portion is approximately 65% to 90% by weight, for example, approximately 70% to 85% by weight; and the proportion of type III collagen to the total collagen in the core portion is approximately 5% to 30% by weight, for example, approximately 10% to 25% by weight (e.g., Fig. 12A and Fig. 12B). In a preferred embodiment of the present invention, in the core portion composed of the fibrous connective tissue, somatic stem cells can be present near the recess, while there is few or no somatic stem cells in a region away from the recess (the center part of the core portion, in particular).

The somatic stem cell-accumulated tissue 23 formed in the recess (the pocket portion) is loose fibrous tissue which contains somatic stem cells, and particularly is enriched in somatic stem cells 23C. In the present invention, the somatic stem cell-accumulated tissue formed in the recess contains type III collagen, and the type III collagen content thereof is larger than the type III collagen content in the core portion. The somatic stem cell-accumulated tissue formed in the recess may also contain other cells, such as fibroblasts or vascular endothelial cells, and it may further contain collagen of other types or other substances. In a preferred embodiment of the present invention, the proportion of type I collagen to total collagen in the somatic stem cell-accumulated tissue is approximately 30% to 75% by weight, for example, approximately 35% to 65% by weight; and the proportion of type III collagen to total collagen is approximately 20% to 65% by weight, for example, approximately 30% to 60% by weight (e.g., Fig. 12C). The amount of fibers in the somatic stem cell-accumulated tissue formed in the recess is small and such fibers are randomly aligned. The collagen fibers are only sparsely present in the somatic stem cell-accumulated tissue 23 formed in the recess. Accordingly, the somatic stem cell-accumulated tissue 23 is loose fibrous soft tissue. Typically, the recess in which the somatic stem cell-accumulated tissue 23 is formed is present on an outer surface of the core portion.

In a preferable embodiment, the proportion of type III collagen to total collagen in the somatic stem cell-accumulated tissue formed in the recess (the pocket portion) of the tissue construct of the present invention is larger than the proportion of type III collagen to total collagen in the core portion of the tissue construct. In an embodiment, the former (i.e., the proportion of type III collagen in the pocket portion) may be 30% to 60% by weight, and the latter (i.e., the proportion of type III collagen in the core portion) may be 10% to 25% by weight.

In a preferred embodiment, the proportion of type I collagen to total collagen in the somatic stem cell-accumulated tissue formed in the recess (the pocket portion) of the tissue construct of the present invention is lower than the proportion of type I collagen to total collagen in the core portion of the tissue construct. In an embodiment, the former (i.e., the proportion of type I collagen in the pocket portion) may be 35% to 65% by weight, and the latter (i.e., the proportion of type I collagen in the core portion) may be 70% to 85% by weight.

The tissue construct according to the present invention, in particular, the core portion and the somatic stem cell-accumulated tissue, do not substantially comprise elastic fiber. The phrase "not substantially comprise elastic fiber " used in the context of the present invention refers to a situation in which no or substantially no elastic fibers are detectable by Elastica van Gieson (EVG) staining.

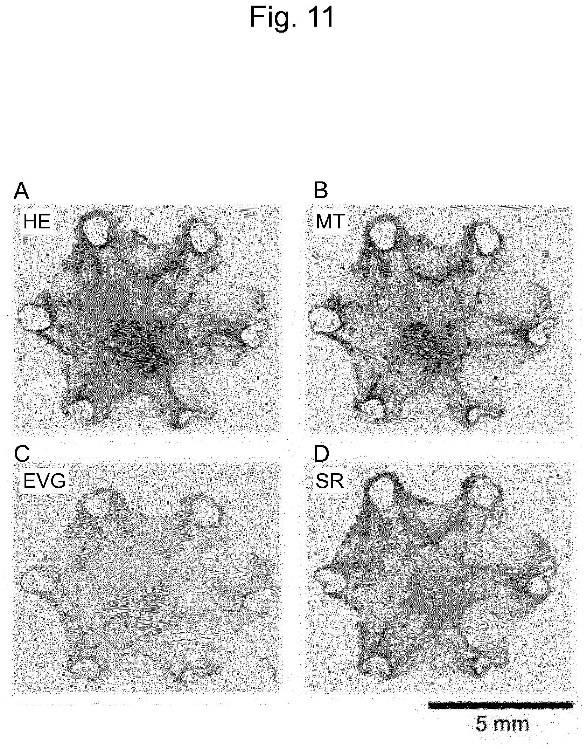

The tissue construct according to the present invention is formed in the hollow portion of the device for producing a tissue construct to fill the hollow portion of the device. Thus, the resulting tissue construct is shaped to fit the form of the hollow portion in the device. When the tissue construct is produced with the use of the device for producing a tissue construct as illustrated in Fig. 4D, for example, the core portion composed of fibrous connective tissue radiates from the center part of the tissue construct toward the surfaces of the six columnar members 13 in accordance with the arrangement of the columnar members 13, and has one recess formed directly below each opening between two columnar members 13 (6 recesses formed in total), and the somatic stem cell-accumulated tissue is formed in each of the 6 recesses, as shown in the cross-sectional photographs of the tissue construct in Fig. 11. Thus, the shape of the tissue construct according to the present invention is defined by the frame body that forms the hollow portion. In other words, the tissue construct according to the present invention is shaped by the device for producing a tissue construct.

In the present invention, the "core portion" composed of the fibrous connective tissue is a core of the tissue construct which supports the somatic stem cell-accumulated tissue formed in the recess of the tissue construct. In a preferred embodiment of the present invention, the core portion continuously extends from the center part of the tissue construct toward the outermost surface of the tissue construct. In the present invention, the "center part" of the tissue construct, if it has a rod-like shape, for example, refers to the full-length of the central axis (the central axis in the longitudinal direction) or a part thereof of the tissue construct and/or a region in the vicinity thereof in the circumferential direction. The central axis is a line that connects center points (the geometric centers) of the transverse cross sections (cross sections orthogonal to the longitudinal direction) of the tissue construct. Alternatively, the "center part" of the tissue construct according to the present invention, if it has a generally polyhedral shape, for example, refers to the full-length of the central axis (the central axis in the longitudinal direction) or a part thereof of the tissue construct and/or a region in the vicinity thereof in the circumferential direction. The central axis is a line that connects center points (the geometric centers) of the transverse cross sections of the tissue construct.

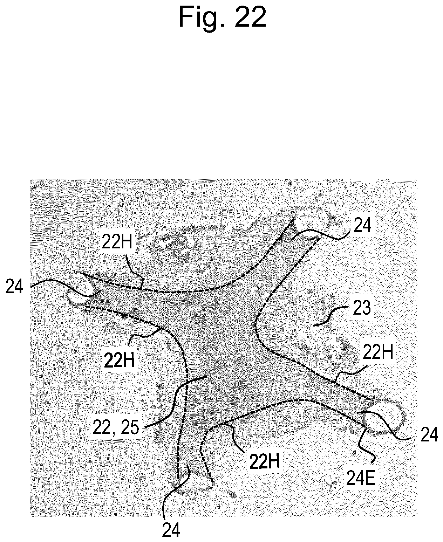

The core portion of the tissue construct according to the present invention preferably has a sectional width of at least 2.5 mm. The "sectional width" of the core portion is as defined above, and it is determined as the longest distance between 2 parallel tangential lines that sandwich a transverse cross section of the core portion. Typically, the sectional width of the core portion may be the longest distance between the edges 24E of two recess peripheries 24 (Fig. 22) that are located in the opposite site of the center part of the core portion, on the transverse cross section of the core portion.

In an embodiment, the tissue construct or the core portion thereof according to the present invention may or may not be solid. The "solid" use in the context of the tissue construct or the core portion thereof means that the tissue construct and the core portion thereof are filled with tissue to the center part thereof. Concerning the tissue construct removed from the device for producing a tissue construct that had been placed in an environment comprising body tissue materials, a thin tissue membrane may also be formed outside of columnar members. However, the presence of such a thin tissue membrane does not indicate that the tissue construct according to the present invention or the core portion thereof is not solid.

The tissue construct according to the present invention has, on the core portion, at least one recess in which the somatic stem cell-accumulated tissue has been formed, and the tissue construct may have preferably 2 or more, and more preferably 3 or more such recesses. The tissue construct according to the present invention may have, on the core portion, for example, 4, 5, 6, 7, 8, 9, or 10 or more recesses in which the somatic stem cell-accumulated tissue is formed. The core portion of the tissue construct according to the present invention may have, for example, 1 to 50, 3 to 50, 4 to 40, 4 to 30, 5 to 30, 3 to 20, 3 to 15, 3 to 10, 4 to 15, or 5 to 15 recesses in which the somatic stem cell-accumulated tissue is formed, per tissue construct. The recess is formed of a fibrous connective tissue. The inner surface of each recess on the core portion is typically configured to have a generally curved surface (e.g., in the form of a mortar).

In the tissue construct according to the present invention, the recess in which the somatic stem cell-accumulated tissue is formed preferably has an opening width WW of at least 2.5 mm, although the opening width is not limited thereto. For calculation, the opening width WW of the recess can be regarded as the same as the opening width W of the opening of the frame body of the device for producing a tissue construct used for the production of the tissue construct. Alternatively, the opening width WW of the recess of the tissue construct according to the present invention can be determined as the shortest distance between the edges (corresponding to the opening edge of the opening of the frame body) on the surface of the frame body (e.g., the columnar member, supporting member, or linear member) of the recess periphery 24 formed at the periphery of the frame body (e.g., the columnar member, supporting member, or linear member) of the device for producing a tissue construct, on the transverse cross section of the tissue construct after the removal of the device for producing a tissue construct. In the tissue construct according to the present invention, the recess on the core portion preferably has an opening configuration with the opening width WW of 2.5 mm or larger, for example, 2.5 mm to 28 mm, 2.5 mm to 25 mm, 2.5 mm to 20 mm, or 3.0 mm to 10 mm.

Alternatively, or in addition to the above, the recess in which the somatic stem cell-accumulated tissue is formed, of the core portion of the tissue construct according to the present invention preferably has an opening configuration (a shape and size) that enables a circle with a diameter of 2.5 mm or larger (e.g., a diameter of 3.0 mm or larger) to be inscribed in the opening. The opening of the recess may have a shape and a size that enable a circle with a diameter of, for example, 2.5 mm to 28 mm, 2.5 mm to 25 mm, 2.5 mm to 20 mm, or 3.0 mm to 10 mm to be inscribed in the opening. The opening of the recess having such shape and size corresponds to the opening configuration of the opening of the frame body that is particularly suitable to form a recess of the fibrous connective tissue and somatic stem cell-accumulated tissue in the recess. The opening of the recess on the core portion of the tissue construct according to the present invention having "an opening configuration that enables a circle to be inscribed in the opening" means an opening where a circle having a given diameter can fit inside the opening configuration (e.g., a rectangular or polygonal configuration) of the opening, in contact with the opening at one or more points. For example, an opening with a rectangular configuration having a longer edge of 20 mm and a shorter edge of 2.5 mm enables a circle with a diameter of up to 2.5 mm to be inscribed therein.

In an embodiment, a fibrous connective tissue or a core portion of the tissue construct according to the present invention may comprise a plurality of the recesses separated by recess peripheries.

In an embodiment, the outer surface of the fibrous connective tissue or the core portion of the tissue construct according to the present invention may be composed of a plurality of the recesses and a plurality of the recess peripheries.

The tissue construct according to the present invention may have any shape such as a rod-like (e.g., a generally cylindrical shape, or generally polygonal prism-like), generally polygonal, generally frustum-like, or generally spherical. In an embodiment, the tissue construct of the present invention may have a rod-like shape, and a plurality of the recesses may be located along the circumferential direction thereof. In another embodiment of the present invention, the tissue construct of the present invention may have a rod-like shape, and a plurality of the recesses may be located along the axial direction thereof. In another embodiment, the tissue construct of the present invention may have a rod-like shape, and a plurality of the recesses may be located along the axial direction and the circumferential direction thereof.

In a further embodiment, the tissue construct of the present invention may have a generally polyhedral shape, and a plurality of the recesses may be located on different faces of the polyhedron.

The tissue construct according to the present invention preferably has a non-tubular shape, and more preferably a non-sheet shape.

The tissue construct according to the present invention has a loose fibrous tissue formed in the recess of the core portion. In the tissue formed in the recess of the tissue construct according to the present invention, somatic stem cells are accumulated (somatic stem cell-accumulated tissue). Somatic stem cells contained in the somatic stem cell-accumulated tissue preferably comprise at least either mesenchymal stem cells or pluripotent stem cells, and more preferably comprise both the mesenchymal stem cells and the pluripotent stem cells. The somatic stem cell-accumulated tissue may comprise stem cells expressing stem cell markers, such as pluripotent stem cells expressing at least 1 pluripotent stem cell marker and/or mesenchymal stem cells expressing at least 1 mesenchymal stem cell marker. The somatic stem cell-accumulated tissue preferably comprises pluripotent stem cells expressing both the pluripotent stem cell markers SSEA4 and SSEA3. The somatic stem cell-accumulated tissue may also comprise stem cells, such as pluripotent stem cells, expressing at least either SSEA3 or SSEA4. Also, the somatic stem cell-accumulated tissue may also comprise mesenchymal stem cells expressing either or both the mesenchymal stem cell markers CD90 and CD105. Also, the somatic stem cell-accumulated tissue may comprise stem cells expressing the mesenchymal stem cell marker CD90, or both CD90 and SSEA3. Also, the somatic stem cell-accumulated tissue may comprise stem cells expressing the mesenchymal stem cell marker CD105, or both CD105 and SSEA3. Also, the somatic stem cell-accumulated tissue may comprise stem cells expressing the growth factor marker VEGF, or both VEGF and SSEA3. Such stem cells have a high angiogenic capacity. The somatic stem cell-accumulated tissue may also comprise stem cells expressing the growth factor marker HGF. The somatic stem cell-accumulated tissue in the tissue construct according to the present invention may comprise somatic stem cells (such as pluripotent stem cells or mesenchymal stem cells) expressing other markers, in addition to or instead of the markers indicated above.

In a preferred embodiment, the tissue construct of the present invention may comprise somatic stem cells (including mesenchymal stem cells and pluripotent stem cells) in a proportion of 1% or more, 5% or more, 10% or more, or 30% or more; and 60% or less, 50% or less, 40% or less, or 30% or less; for example, 1% to 60%, 5% to 40%, 5% to 30%, or 5% to 20%, relative to the total number of cells contained in the tissue construct (the proportion of stem cells based on the number of cells).

In a preferred embodiment, the somatic stem cell-accumulated tissue of the present invention may comprise somatic stem cells (including mesenchymal stem cells and pluripotent stem cells) in a proportion of 20% or more, 30% or more, or 50% or more; and 90% or less, 80% or less, or 70% or less; for example, 20% to 90%, 30% to 90%, 30% to 80%, or 50% to 90%, relative to the total number of cells contained in the somatic stem cell-accumulated tissue (the proportion of stem cells based on the number of cells).

In a preferred embodiment, when the tissue construct and/or the somatic stem cell-accumulated tissue of the present invention are subjected to a treatment in a 0.25% collagenase type I solution at 37°C for 1.5 hours, as described in Example 4, the cells collected from the tissue degraded as a result of such treatment (i.e., the somatic stem cell-accumulated tissue and its vicinity) may comprise somatic stem cells (including mesenchymal stem cells and pluripotent stem cells) in a proportion of 20% or more, 30% or more, or 50% or more; and 90% or less, 80% or less, or 70% or less; for example, 20% to 90%, 30% to 90%, 30% to 80%, or 50% to 90%, relative to the total number of the collected cells (the proportion of stem cells based on the number of cells).

The above-mentioned proportion of stem cells can be calculated with the use of the number of cells expressing at least one stem cell marker (e.g., at least one marker selected from the group consisting of CD90, CD105, SSEA3, and SSEA4, or at least one marker selected from the group consisting of CD90, SSEA3, and SSEA4, although the marker is not limited thereto) as the number of stem cells (that is, the number of somatic stem cells).

In an embodiment, the tissue construct and the somatic stem cell-accumulated tissue of the present invention can comprise mesenchymal stem cells and pluripotent stem cells at a ratio of mesenchymal stem cells to pluripotent stem cells of 60 to 95:5 to 40; 60 to 80:10 to 30; or 65 to 75:15 to 25, although the ratio is not limited thereto. The ratio of mesenchymal stem cells to pluripotent stem cells can be calculated with the use of the number of cells expressing at least one mesenchymal stem cell marker (e.g., CD90 or CD105, although the marker is not limited thereto) as the number of mesenchymal stem cells, and the number of cells expressing at least one pluripotent stem cell marker (e.g., SSEA3 or SSEA4, although the marker is not limited thereto) as the number of pluripotent stem cells.

The somatic stem cell-accumulated tissue formed in the recess in the tissue construct according to the present invention comprises, in addition to the somatic stem cells as described above, type III collagen. The somatic stem cell-accumulated tissue may further comprise other cells, such as fibroblasts or vascular endothelial cells, collagen of other types, or other substances. The presence of vascular endothelial cells can be examined by detecting the expression of, for example, the vascular endothelial cell marker vWF. The presence of fibroblasts can be examined by detecting the expression of, for example, the fibroblast marker vimentin.

In the tissue construct according to the present invention, a high-level expression of VEGF can be observed particularly in a region on the relatively outer side. On the other hand, in fibroblasts that are contained at a high level in the center part of the tissue construct according to the present invention, an expression level of VEGF is typically low.

The tissue construct according to the present invention may be one formed by placing the device for producing a tissue construct in an environment comprising body tissue materials, such as in body tissue in a living body.

The tissue construct according to the present invention may be in a state as it remains in the device after the production of the tissue construct in an environment comprising body tissue materials with the use of the device for producing a tissue construct. In that case, it is preferred that the tissue construct fill the hollow portion in the device for producing a tissue construct and the core portion be adhered to the surface of the frame body (e.g., the columnar member, the supporting member, and/or the linear member) of the device. After the tissue construct according to the present invention is formed, alternatively, it may be separated from the device for producing a tissue construct.

The somatic stem cell-accumulated tissue formed in the recess of the fibrous connective tissue (the core portion) of the tissue construct according to the present invention can be easily collected separately from the fibrous connective tissue. For example, the somatic stem cell-accumulated tissue 23 can be easily scraped from the recess 22H of the fibrous connective tissue 22 (the core portion 25) of the tissue construct according to the present invention. Alternatively, the somatic stem cell-accumulated tissue 23 or the somatic stem cells 23C can be collected from the device 10 removed from the environment comprising body tissue materials, such as body tissue in a living body, by scraping, from the recess 22H, the somatic stem cell-accumulated tissue 23 comprising the accumulated somatic stem cells 23C or enzymatic degradation of the somatic stem cell-accumulated tissue 23. The device 10 removed from the environment comprising body tissue materials may be directly subjected to enzymatic treatment with a degrading enzyme, such as collagenase, elastase, trypsin, TrypLE^{™} (Thermo Fisher Scientific), Accutase^{®} (Innovative Cell Technologies, Inc.), or Dispase, to separate the somatic stem cells 23C from the somatic stem cell-accumulated tissue 23 formed in the recess in the hollow portion of the device. Alternatively, the somatic stem cell-accumulated tissue 23 scraped from the recess 22H may be subjected to enzymatic treatment with a degrading enzyme, such as collagenase, elastase, trypsin, TrypLE^{™} (Thermo Fisher Scientific), Accutase^{®} (Innovative Cell Technologies, Inc.), or Dispase, to separate the somatic stem cells 23C from the somatic stem cell-accumulated tissue 23. While the duration of enzymatic treatment depends on the amount of samples to be treated, typically, it may be approximately 0.5 to 2 hours. The cells survived after the enzymatic treatment may be separated from the fibrous connective tissue 22, and the somatic stem cells 23C may be collected therefrom.

A further step of isolation and purification may be performed to enhance the purification level of the somatic stem cells 23C. For example, a filtration treatment may be performed with the use of a membrane filter or a mesh filter to separate cells comprising the somatic stem cells 23C. The somatic stem cells 23C can be isolated and/or purified with the use of markers specifically expressed on the surfaces of somatic stem cells, such as stem cell markers (e.g., the pluripotent stem cell markers and/or the mesenchymal stem cell markers). For example, either or both the pluripotent stem cell markers SSEA4 and SSEA3, or either or both the mesenchymal stem cell markers CD105 and CD105 can be used as stem cell markers. Somatic stem cells can be easily separated and/or concentrated from the somatic stem cell-accumulated tissue, in accordance with a conventional technique including a cell sorting technique, such as magnetic cell sorting (MACS) or fluorescence-activated cell sorting (FACS).

The present invention also relates to a method for collecting somatic stem cells 23C comprising separating somatic stem cell-accumulated tissue or somatic stem cells from the tissue construct according to the present invention.

The somatic stem cells isolated from the somatic stem cell-accumulated tissue in the tissue construct according to the present invention may or may not be cultured and proliferated as appropriate. Alternatively, a cell population comprising the somatic stem cell-accumulated tissue or somatic stem cells partially purified from the somatic stem cell-accumulated tissue may or may not be cultured and proliferated as appropriate. The somatic stem cell-accumulated tissue, the cell population comprising the somatic stem cells, or the somatic stem cells can be used for tissue construction or cell differentiation *in vitro* or *ex vivo.* The somatic stem cell-accumulated tissue, the cell population comprising the somatic stem cells, or the somatic stem cells can be used, by administration to a subject (e.g., a patient), for regenerative medicine, such as tissue repair (tissue regeneration), prevention or treatment (e.g., repairment or improvement) of impairment (e.g., congenital or acquired defect and congenital or acquired dysfunction, malfunction, or damage) or functional deterioration of tissue or organs; or functional improvement of tissue or organs, by administering them to a target (e.g., a patient) and used for tissue engineering. Specifically, a cell-based medicine comprising the somatic stem cell-accumulated tissue, a cell population comprising the somatic stem cells, or the somatic stem cells obtained in the present invention may be used for regenerative therapies, such as tissue repair (tissue regeneration), prevention or treatment (e.g., repairment or improvement) of impairment or functional deterioration of tissue or organs, or functional improvement of tissue or organs. The present invention also provides a cell-based medicine (or a pharmaceutical composition) comprising the somatic stem cell-accumulated tissue obtained from the tissue construct according to the present invention, the somatic stem cells derived from the somatic stem cell-accumulated tissue, or a cell population comprising the somatic stem cells. The present invention also provides a method for producing a cell-based medicine (or a pharmaceutical composition) comprising preparing a cell-based medicine (or a pharmaceutical composition) using the somatic stem cell-accumulated tissue obtained from the tissue construct according to the present invention, the somatic stem cells derived from the somatic stem cell-accumulated tissue, or the cell population comprising the somatic stem cells. The cell-based medicine or pharmaceutical composition according to the present invention may further comprise a pharmaceutically acceptable additive which includes, but are not limited to, carrier, solvent, excipient, moistening agent, stabilizing agent, tonicity agent, buffer, preservative, coloring agent, and cryoprotective agent.

The somatic stem cell-accumulated tissue obtained in the present invention, the cell population comprising the somatic stem cells, or the somatic stem cells may be differentiated into cells of interest by induction outside body tissue, followed by administration to a subject (e.g., a patient); or may be administered to an appropriate body tissue (e.g., a site of tissue or an organ with impairment or functional deterioration) without differentiation induction. The somatic stem cell-accumulated tissue obtained in the present invention, the cell population comprising the somatic stem cells, or the somatic stem cells can be cryopreserved in accordance with a conventional technique and can be administered when necessary. Differentiation induction may be performed in accordance with a conventional technique for cell differentiation induction or any known method. The present invention also provides a cell-based medicine (or a pharmaceutical composition) comprising the somatic stem cell-accumulated tissue of the present invention, the somatic stem cells derived from the somatic stem cell-accumulated tissue, or a cell population comprising the somatic stem cells, which have been subjected to the differentiation induction treatment; such as cells differentiated from the somatic stem cells derived from the somatic stem cell-accumulated tissue according to the present invention. The present invention also provides a method for producing a cell-based medicine (or a pharmaceutical composition) comprising preparing a cell-based medicine (or a pharmaceutical composition) using the somatic stem cell-accumulated tissue of the present invention, the somatic stem cells derived from the somatic stem cell-accumulated tissue, or the cell population comprising the somatic stem cells, which have been subjected to the differentiation induction treatment. The cell-based medicine or pharmaceutical composition may further comprise a pharmaceutically acceptable additive which includes, but are not limited to, carrier, solvent, excipient, moistening agent, stabilizing agent, tonicity agent, buffer, preservative, coloring agent, and cryoprotective agent.

The present invention also relates to a method for providing a regenerative therapy to a subject (e.g., a patient), a method of prevention or treatment (e.g., repairment or improvement) of impairment or functional deterioration of tissue or organ, or a method of functional improvement of tissue or organs, the method comprising administering the somatic stem cell-accumulated tissue according to the present invention, the somatic stem cells derived from the somatic stem cell-accumulated tissue, the cell population comprising the somatic stem cells, or cells differentiated from the somatic stem cells to the subject. Examples of the subject (e.g., patient) include, but are not limited to, mammalian animals, including primate, such as human, monkey, and chimpanzee,, and dog, cat, cow, pig, horse, goat, sheep, rat, and mouse; birds, fish, and amphibians. In an embodiment, the subject (e.g., patient) may be human, or non-human animals, such as non-human mammalian animals. In an embodiment, the subject (e.g., patient) may be in need of a regenerative therapy, such as tissue repair (tissue regeneration), prevention or treatment (e.g., repairment or improvement) of impairment or functional deterioration of tissue or organs, or functional improvement of tissue or organs. The subject (e.g., patient) may have impairment or functional deterioration of tissue or organs, which can be expected to be cured via implantation of somatic stem cells or cells differentiated from somatic stem cells.

According to the present invention, a tissue construct comprising somatic stem cells accumulated therein can be produced, somatic stem cells can be efficiently collected, and somatic stem cells become easily obtained and used.

### Examples

Hereafter, the present invention is described in greater detail with reference to the following Examples, although the technical scope of the present invention is not limited to these Examples.

### [Materials and Equipment]

In the Examples of this description, the test animal, the reagents, and the devices indicated below were used.
- Test animal: Beagle dogs, female, 3-year-old, body weights of 10 kg
- The devices for producing a tissue construct illustrated in Figs. 4 to 6
- The stent having the configuration illustrated in Fig. 9 and a balloon catheter
- Tissue preservation medium HypoThermosol^{®} FRS (BioLife Solutions, 101102)
- Collagenase, Type I (Worthington, LS004214)
- Dulbecco's Modified Eagle Medium (DMEM) (DMEM-LG (low glucose), Wako, 041-29775)
- Cell cryopreservation medium Bambanker^{®} (NIPPON Genetics Co, Ltd., CS-02-001)
- 4% Paraformaldehyde (PFA, Wako, 163-20146)
- Hemolytic reagent: VersaLyse Lysing Solution (Beckman Coulter, A09777)
- Fetal Bovine Serum (FBS) (Biosera, FB-1365)
- Phosphate Buffered Saline (PBS)
- Xylene (Muto Pure Chemicals Co., Ltd, 4313)
- 100% Ethanol (Muto Pure Chemicals Co., Ltd., 4026)
- Mordant (Muto Pure Chemicals Co., Ltd., 4006-1)
- Second mordant (Muto Pure Chemicals Co., Ltd., 8141-1)
- Weigert's iron hematoxylin solution 1 (Muto Pure Chemicals Co., Ltd., 4034-1)
- Weigert's iron hematoxylin solution 2 (Muto Pure Chemicals Co., Ltd., 4035-1)
- 0.75% Orange G (Muto Pure Chemicals Co., Ltd., 4023-1)
- 1% Acetic acid (Muto Pure Chemicals Co., Ltd., 4017-1)
- Masson staining solution B (Muto Pure Chemicals Co., Ltd., 4025-1)
- 2.5% Phosphotungstic acid solution (Muto Pure Chemicals Co., Ltd., 4018-1)
- Aniline Blue Solution (Muto Pure Chemicals Co., Ltd., 4020-1)
- Maeda's modified resorcin-fuchsin solution (Muto Pure Chemicals Co., Ltd., 4032-1)
- Van Gieson Solution A (Muto Pure Chemicals Co., Ltd., 4036-1)
- Van Gieson Solution B (Muto Pure Chemicals Co., Ltd., 4037-1)
- 1% Sirius Red Solution (Muto Pure Chemicals Co., Ltd., 3306-1)
- Citrate buffer (Sigma, C9999-1000ML)
- Bovine Serum Albumin (BSA) (Wako, 013-25773)

### < Primary antibodies>

- Anti-CD90 antibody (Abcam, ab123511)
- Anti-CD105 antibody (Abcam, ab156756)
- Anti-SSEA4 antibody (Abcam, ab16287)
- Anti-VEGFA antibody (Abcam, ab1316)
- Anti-HGF antibody (Abcam, ab83760)
- Anti-SSEA3 antibody (Bioss, bs-3575R)
- Anti-Von Willebrand Factor (vWF) antibody (Abcam, ab6994)
- Anti-vimentin antibody (Abcam, ab8069)

### <Secondary antibody (fluorescently labeled)>

- Goat anti-mouse IgG H&L (Alexa Fluor^{®} 488) (Abcam, ab150113)
- Goat anti-rabbit IgG H&L (Alexa Fluor ^{®} 594) (Abcam, ab150080)
- ProLong^{™} Gold Antifade Mountant with DAPI (4',6-diamidino-2-phenylindole)

### (Thermo Fisher Scientific, P36931)

- Cell Staining Buffer (BioLegend, 420201)
- FITC-labeled anti-CD90 antibody (BioLegend, 328107)
- Anti-FITC microbeads (Miltenyi Biotec, 130-048-700)
- Goat anti-rabbit IgG microbeads (Miltenyi Biotec, 130-048-600)
- MACS buffer (autoMACS^{®} Rinsing Solution, Miltenyi Biotec, 130-091-222)
- MACS separation column (MS Column, Miltenyi Biotec, 130-042-201)

- Fluorescent microscope (Nikon, ECLIPSE Ti-U)
- Polarizing microscope (Nikon, ECLIPSE E1000)

### [Example 1] Production of tissue construct via in-body tissue architecture (iBTA)

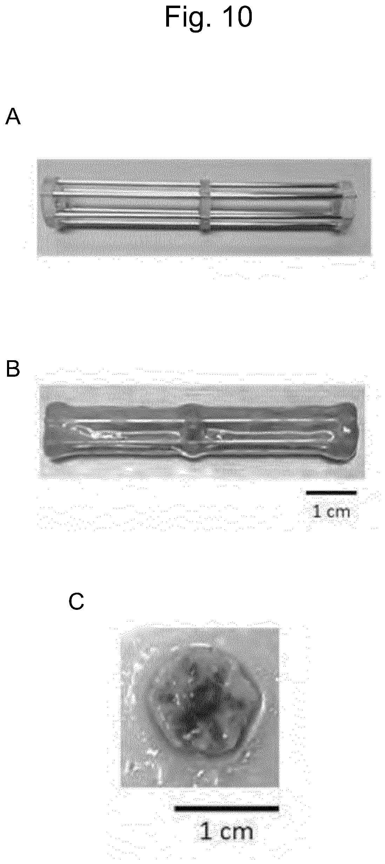

A beagle dog was anesthetized, the skin was incised with an electrosurgical knife at 5 sites from the right lateroabdominal region to the back, one device for producing a tissue construct having the configuration illustrated in Fig. 4D and Fig. 10A was subcutaneously inserted and implanted per each incised site, and then the wounds were sutured. After placing the devices in a living body for 3 weeks, the beagle dog was anesthetized, the skin was incised, and the subcutaneously-implanted devices for producing a tissue construct were removed. The used devices for producing a tissue construct comprise 6 circular cylindrical columnar members 13 having a diameter of 1 mm and a length of 60 mm which are fixed at both ends and at the intermediate parts of the columnar members with ring-shaped supporting members having a diameter of 11 mm. Regarding the devices used herein, the opening width W is 4.0 mm, and the depth D is 4.6 mm.

The device removed from the subcutaneous region was almost entirely covered with soft tissue. The cross section of the device was observed and the device was found to be filled with tissue to the center of the hollow portion (hollow space) of the device. It was considered that cells were accumulated in the hollow portion of the device for producing a tissue construct that had been implanted and placed in the living body, and tissue formation occurred as a result. A difference in the site of implantation did not substantially affect tissue formation.

Fig. 10 shows exemplary photographs of the device used (A), the device removed 3 weeks after implantation (B) in a living body, and the cross section of the resulting tissue construct (C).

It was shown that a tissue construct could be produced by implanting and placing the device for producing a tissue construct according to the present invention *in vivo.*

The device for producing a tissue construct removed from the subcutaneous region was transferred into a 15-ml centrifuge tube containing a tissue preservation medium HypoThermosol^{®} FRS, placed on ice, stored at 4°C, and subjected to the subsequent treatment within 24 hours thereafter.

In a clean bench, the device for producing a tissue construct having the tissue construct formed therein was removed from the tissue preservation medium, the device was gently washed in a centrifuge tube containing PBS, and excess tissue around the device was trimmed away. Thereafter, the device was photographed and stored in DMEM.

### [Example 2] Histological analysis of tissue construct

The device for producing a tissue construct removed from the living body in Example 1 was treated with 4% PFA at 4°C for 24 hours to fix tissue, and the device was then detached from the tissue construct and a paraffin-embedded block of the tissue construct was prepared.

The paraffin-embedded block was transversely cut with a microtome to prepare sections with thickness of 3 to 5 µm. The sections were subjected to hematoxylin-eosin (HE) staining, Masson trichrome (MT) staining, Elastica van Gieson (EVG) staining, and Sirius Red (SR) staining, and then observed under the microscope.

As a result of staining, the tissue construct was stained generally pink via HE staining (Fig. 11A) and blue via MT staining (Fig. 11B). This indicates that the tissue construct obtained is rich in collagen fibers. In addition, the tissue construct was stained generally red via SR staining. This indicates that the tissue construct obtained is rich in collagen (Fig. 11D). In the tissue construct, in particular, a region from around (the vicinity of) the columnar members of the device for producing a tissue construct to the center part of the tissue construct was deeply stained. This indicates that collagen is present densely in the region. Thus, it was revealed that fibrous connective tissue was formed around the columnar members and at the center part of the tissue construct. On the other hand, there were regions which are only weakly stained with all of HE, MT, and SR staining in the tissue construct, and are between the columnar members. This indicates that collagen fibers were very sparsely (loosely) present in the regions. It was shown that the connective tissue formed around the columnar members and in the center part of the tissue construct had recesses having a generally curved surface as formed between the columnar members, and soft tissue (loose fibrous tissue) corresponding to the weakly-stained region ("pocket portion") was formed in the recesses.

No region was stained black with EVG staining. This indicates that the tissue construct comprises substantially no elastic fibers (Fig. 11C).

In order to determine the type of collagen present in the tissue construct, the sections subjected to Sirius Red (SR) staining were observed under a polarizing microscope. In polarizing microscopy, type I collagen is observed as yellow to orange, and type III collagen is observed as green.

As a result of observation, densely aligned collagen fibers were observed as yellow or orange in a region deeply stained with Sirius Red (i.e., around the columnar members of the device and the center part of the tissue construct). This indicates that type I collagen is mainly present in such region (Fig. 12A and 12B). Meanwhile, weakly-stained regions between the columnar members were observed as green. This indicates that the amount of type III collagen in such region is larger than a region around the columnar member of the device and at the center part of the tissue construct (Fig. 12C). The amount of fibers contained in the weakly-stained regions (pocket portions) between columnar members was small, and such fibers were randomly aligned therein (Fig. 12C).

### [Example 3] Immunohistochemical staining

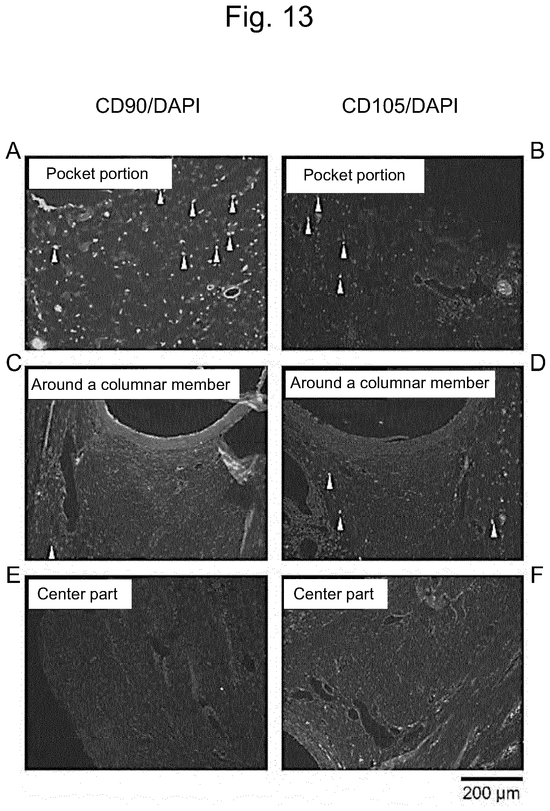

In order to determine the types of cells present in the tissue construct and the distribution thereof, immunohistochemical staining of the tissue construct was performed. Sections of the tissue construct with thickness of 3 to 5 µm were subjected to immunohistochemical staining to detect the mesenchymal stem cell markers CD90 and CD105, the pluripotent stem cell markers SSEA4 and SSEA3, the vascular endothelial growth factor (VEGF), and the hepatic cell growth factor (HGF) with the use of primary antibodies and secondary antibodies against them. In addition, cell nuclear staining was performed by the treatment with DAPI (4',6-diamidino-2-phenylindole). The results are shown in Figs. 13 to 15.

In the staining for detecting the mesenchymal stem cell markers CD90 and CD105, many cells were stained in the pocket portions. Even around the columnar members, positive cells were observed near the pocket portions. In Fig. 13, representative stained sites are indicated using arrowheads. There were no positive cells in the center part of the tissue construct.

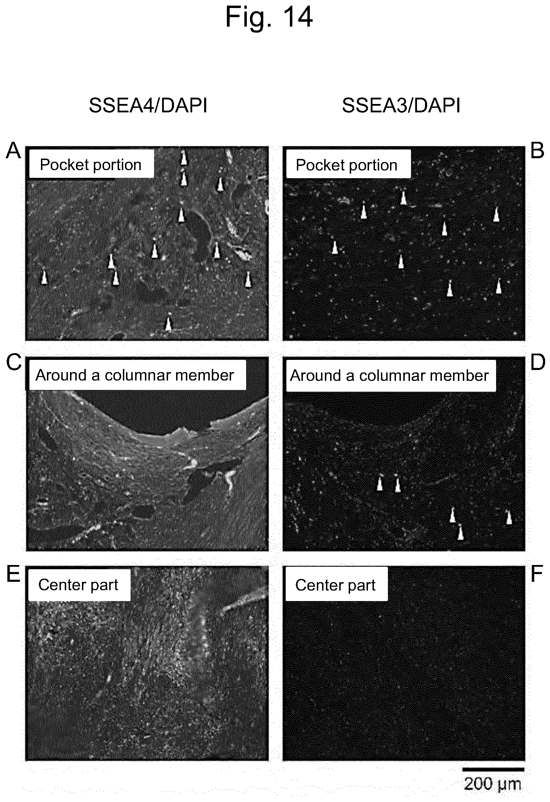

In the staining for detecting the pluripotent stem cell markers SSEA4 and SSEA3, many cells were stained in the pocket portions, as in the case of the mesenchymal stem cell markers. In Fig. 14A and 14B, representative stained sites are indicated using arrowheads. There were no positive cells in the center part of the tissue construct.

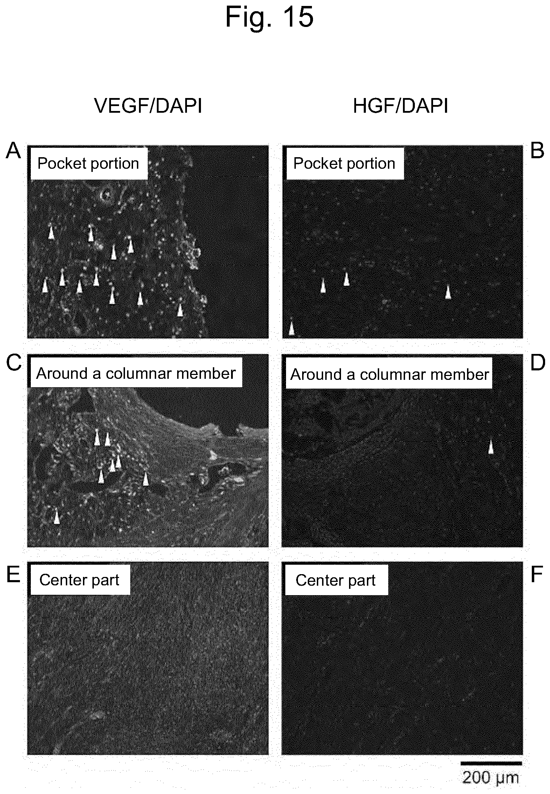

Regarding the expression inside the tissue construct, the VEGF expression level was high in a region on the relatively outer side of the entire tissue construct, and the VEGF expression level was particularly high in round cells in the pocket portions (Fig. 15). Many HGF-expressing cells were observed in the pocket portions, and the HGF expression level was high in round cells, as in the case of VEGF (Fig. 15). In general, round cells are considered to be highly undifferentiated. In contrast, the VEGF expression level was low in fibroblasts.

In order to examine properties of the stem cells, dual staining for the stem cell marker SSEA3 and other markers (CD90 and SSEA3; CD105 and SSEA3; SSEA4 and SSEA3; and VEGF and SSEA3) was performed.

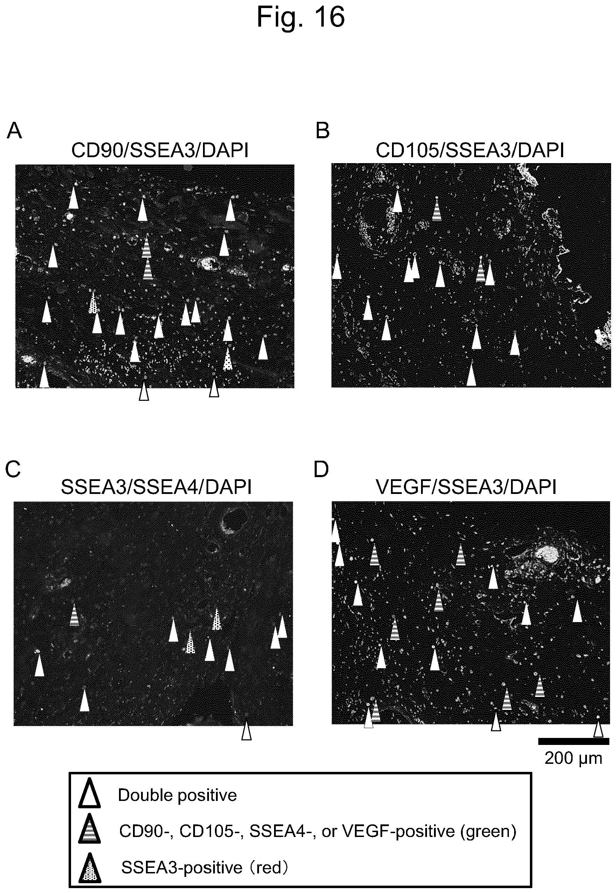

As a result of dual staining, double positive cells (indicated by white arrowheads) as well as cells positive for a single marker (indicated by horizontal-striped arrowheads and dotted arrowheads) were observed (Fig. 16). Horizontal-striped arrowheads indicate a green staining being CD90-, CD105-, SSEA4-, or VEGF-positive. Dotted arrowheads indicate a red staining being SSEA4-positive. The cells which are components of the tissue in the pocket portions were found to be a differentially undifferentiated and hierarchical cell population. Some cells thereof were found to simultaneously express the pluripotent stem cell markers SSEA4 and SSEA3 (Fig. 16C), which was consistent with the features of more highly undifferentiated pluripotent stem cells, such as ES cells (Henderson J. K., et al., Stem cells, 2002; 20: 329-337). In addition, the VEGF expression level was particularly high in SSEA3-positive cells (Fig. 16D). Thus, it is considered that highly undifferentiated stem cells present in the tissue in the pocket portions have a high angiogenic capacity. In fact, immunohistochemical staining was performed with the anti-vWF antibody, and the expression of vascular endothelial cell marker vWF was highly detected in a region mainly including the boundary between the pocket portions and the core portion, which indicates the presence of vascular endothelial cells near the boundary and in the pocket portions. Further, immunohistochemical staining was performed with the anti-vimentin antibody, and the expression of fibroblast marker vimentin was also detected in the pocket portions.

These results mentioned above demonstrate that stem cells are accumulated and activated in the pocket portions, so as to promote tissue formation. That is, the tissue (pocket portion) formed in the recess is a tissue in which somatic stem cells are accumulated ("somatic stem cell-accumulated tissue"). The production of a tissue construct comprising such pocket portion enables activated stem cells to be efficiently corrected and available.

### [Example 4] Cell separation and analysis

DMEM (serum-free; 20 ml) was added to 50 mg of collagenase, type I in a vial to prepare a 0.25% collagenase solution. The device for producing a tissue construct comprising the tissue construct formed therein, which was removed from the living body in Example 1, was transferred into a 15-ml centrifuge tube containing 6 ml of the collagenase solution prepared, and the tube was incubated at 37°C with shaking in an incubator. After the incubation with shaking for 1.5 hours, the device and the tissue undegraded with collagenase were removed away. The centrifuge tube was centrifuged at 4°C and 1,000 rpm for 5 minutes and the supernatant was removed. DMEM (6 ml) was added, pipetted, and then cells were collected into 50-ml centrifuge tubes (4 tubes, 24 ml). The collected fraction was centrifuged at 4°C and 1,000 rpm for 5 minutes and the supernatant was removed.

In order to hemolyze the erythrocytes, 1 ml of the VersaLyse Lysing Solution was added to the collected cells, pipetted, and the cells were incubated at room temperature for 10 minutes. DMEM supplemented with 10% FBS (10 ml) was added, the resultant was centrifuged at 4°C and 1,000 rpm for 5 minutes, and the supernatant was removed. The cell cryopreservation medium Bambanker^{®} (5 ml) was added thereto to prepare a suspension, 10 µl of the cell suspension was sampled, an equal volume of a trypan blue stain solution was added for staining, and the number of stained cells was counted. The cell suspension was divided into 1 ml aliquots in cryotubes and stored in a liquid nitrogen tank.

The cell concentration of the 5 ml suspension in the Bambanker^{®} was 6.25 × 10⁶ cells/ml, and the cell viability was 98.4%. Thus, approximately 4 × 10⁶ cells were collected per tissue construct. Also in the other experiments performed independently, comparable number of cells were collected. Under the treatment conditions of this Example, substantially the whole somatic stem cell-accumulated tissue and the connective tissue in the vicinity thereof (i.e., the peripheral region of the tissue construct) were degraded; however, the center part of the tissue construct (in particular, that of the core portion) was not degraded.

In order to determine the cell abundance ratio in the collected cell population, the CD90-positive cells, the SSEA3-positive cells, and the SSEA4-positive cells were counted using a flow cytometer.

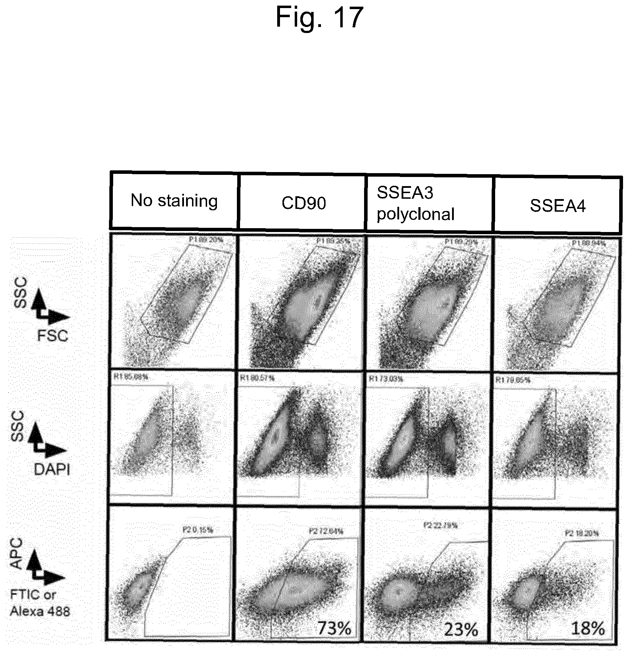

As a result, the percentage of the CD90-positive cells was 73%, that of the SSEA3-positive cells was 23%, and that of the SSEA4-positive cells was 18% in the collected cell population (Fig. 17). The percentage of cells expressing stem cell markers was approximately 80% in the collected cell population. On the basis of the marker expression, approximately 70% of the cells expressing stem cell markers was mesenchymal stem cells (2.8 × 10⁶ cells) and approximately 20% was pluripotent stem cells (8.0 × 10⁵ cells).

Upon collagenase treatment, degradation of the tissue construct begins from the outer side and cells are separated therefrom. Accordingly, most cells separated at the early stage of collagenase treatment were somatic stem cells derived from the somatic stem cell-accumulated tissue. It was shown that the proportion of fibroblasts would increase depending on the duration of collagenase treatment.

The amount of the mesenchymal stem cells contained in bone marrow is as small as 10 to 100 cells/ml, and that in adipose tissue is as small as 5 × 10³ cells/g, both of which are common sources of mesenchymal stem cells. Since the cells from the tissue construct according to the present invention contain stem cells at a high proportion, the present invention enables stem cells to be collected with higher efficiency, compared with a conventional method for stem cell collection.

### [Example 5] Concentration of CD90- or SSEA3-positive cells

From the cell population collected from the tissue construct subjected to collagenase treatment in the same way as in Example 4, CD90- or SSEA3-positive stem cells were separated and concentrated. Specifically, the collected cell population was labeled with anti-CD90 antibody (BioLegend, 328107) or anti-SSEA3 antibody (Bioss, bs-3575R), reacted with magnetic bead-conjugated antibodies: anti-FITC MicroBeads (Miltenyi Biotec, 130-048-700) and goat anti-rabbit IgG MicroBeads (Miltenyi Biotec, 130-048-600), and then subjected to the cell separation via magnetic cell sorting (MACS).

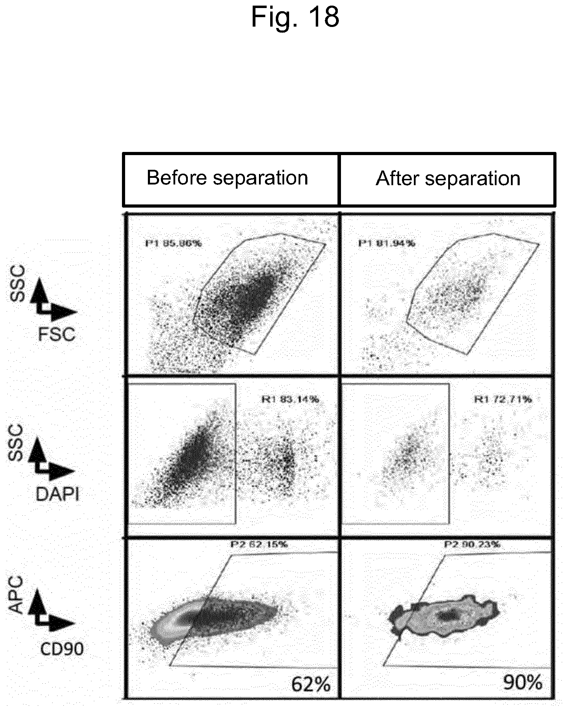

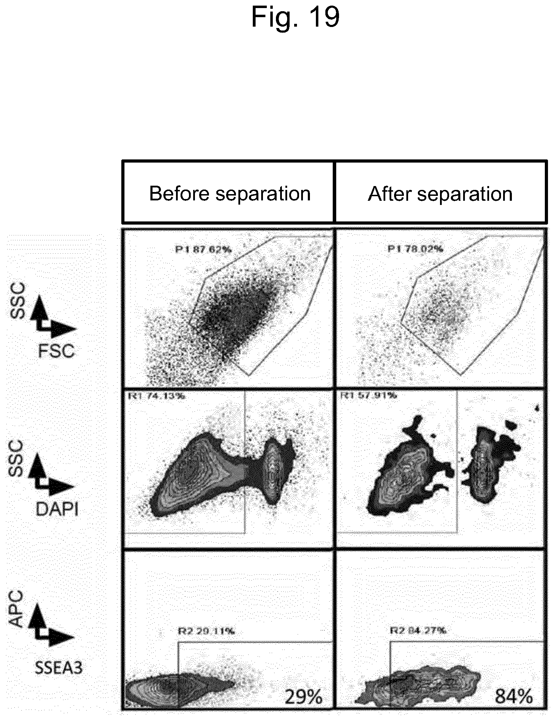

As a result, CD90-positive cells were concentrated from 62% (before separation) to 90% (after separation) in the cell proportion (Fig. 18). Also, SSEA3-positive cells were concentrated from 29% (before separation) to 84% (after separation) in the cell proportion (Fig. 19).

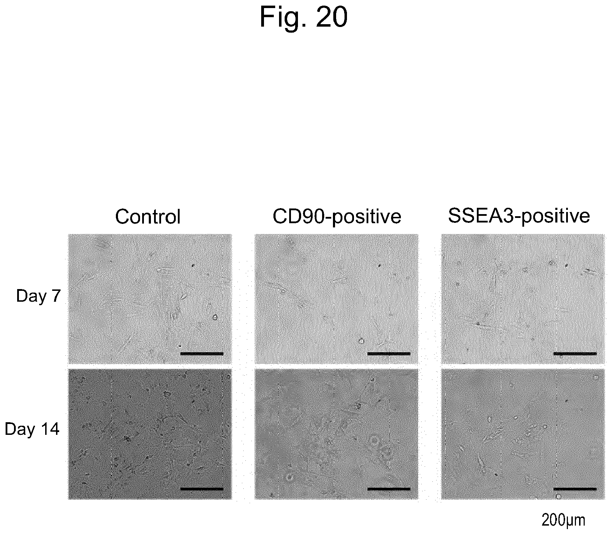

The cell population before cell separation via MACS (control) and the cell population after the cell separation were seeded on a 6-well plate at a density of 3,000 cells/cm², cultured in DMEM supplemented with 10% FBS, and the cell morphology was observed. As a result, fibroblast-like cells were found to have adhered to the bottom of the plate and have proliferated (Fig. 20). While the cells separated with the aid of the stem cell marker had a fibroblast-like morphology, smaller cells were more frequently observed, compared with the cell population before separation (Fig. 20).

As such, with the use of the tissue construct according to the present invention as a cell source, stem cells of interest could be concentrated efficiently.

### [Example 6]

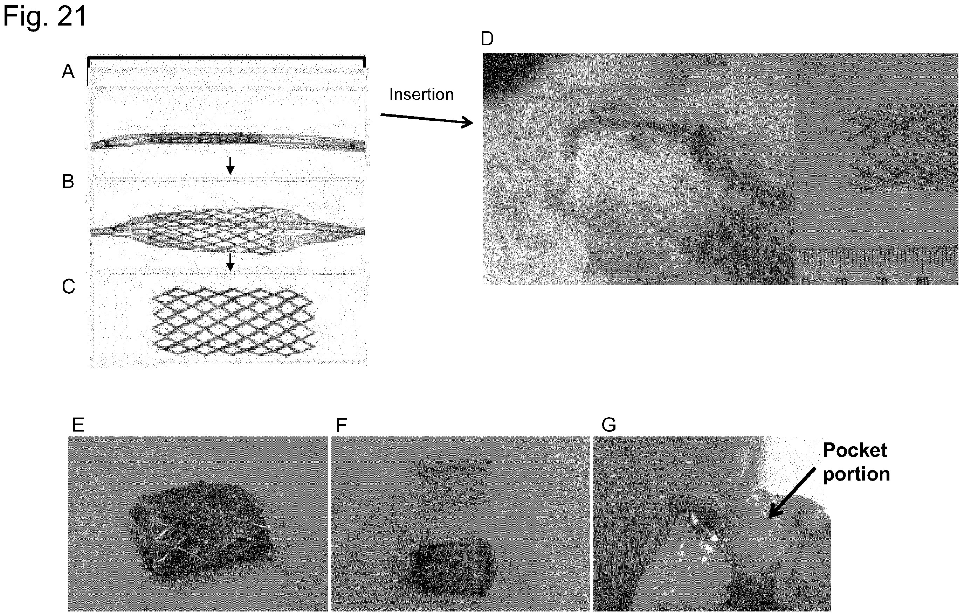

The skin of a beagle dog was punctured and then a balloon catheter with a closed stent (having a length of 29 mm and a diameter of 20 mm, in the expanded state) mounted thereon was inserted thereinto subcutaneously (Fig. 21A and 21D). The balloon was inflated subcutaneously by applying hydraulic pressure (Fig. 21B), to cause the stent to expand (Fig. 21C).

The hydraulic pressure was released, the balloon was deflated and the catheter was withdrawn, thereby subcutaneously placing the stent in the expanded state. The beagle dog was anesthetized 3 weeks later, the skin was incised, and the stent placed subcutaneously was isolated.

The stent was found to be filled with tissue to the center part (Fig. 21E). The tissue construct formed in the stent was removed (Fig. 21F). Fibrous connective tissue was formed around metal frames of the stent and at the center part of the tissue construct, and the connective tissue formed recesses having a curved surface between metal frames adjacent to each other. The pocket portion comprising stem cells accumulated therein (somatic stem cell-accumulated tissue) was formed in the recess (Fig. 21G, indicated with an arrow). Also at each of the pass-through openings 12H at the both ends of the stent, the recess comprising the somatic stem cell-accumulated tissue formed therein were observed, as in the case between metal frames. As such, it was shown that the tissue construct according to the present invention could also be produced with the use of a stent having the configuration illustrated in Fig. 9.

### [Example 7]

The devices for producing a tissue construct according to the present invention with various configurations were used to produce tissue constructs. Five types of devices for producing a tissue construct 10 having the configurations illustrated in Fig. 4A to 4C and Fig. 5A and 5B, respectively, were prepared. The 5 types of the devices for producing a tissue construct used include columnar members 13 each having a circular cylindrical shape with a diameter of 1 mm. Under sufficient anesthesia, a beagle dog was incised, the device for producing a tissue construct was subcutaneously implanted in the abdominal region, and the wound was sutured. After 3 days, 10 days, 2 weeks (14 days), 17 days, or 3 weeks (21 days) of the implantation of the device, the beagle dog was incised to remove the device for producing a tissue construct from the abdominal subcutaneous region.

The device for producing a tissue construct removed from the subcutaneous region was almost entirely covered with soft tissue, and the hollow portion (hollow space) of the device was filled with tissue. As with the above-mentioned Examples, formation of the tissue construct was observed.

The tissue construct removed from the device was subjected to fiber staining to stain collagen fibers in the same way as in Example 2. As one example thereof, Fig. 22 shows an HE staining image of a section located around the center of the tissue construct along with the longitudinal direction which was formed with the use of the device for producing a tissue construct illustrated in Fig. 4B (the opening width W of the opening: 6.0 mm). The results very similar to those in Example 2 were shown. Connective tissue was present in a region from around each columnar member to the center part of the tissue construct, the connective tissue formed the recess 22H between the columnar members, and the loose fibrous, somatic stem cell-accumulated tissue (the pocket portion) 23 was present in the recess. The connective tissue was mainly composed of dense collagen fibers.

On the basis of the result of tissue analysis of the tissue constructs, it was found that the formation of fibrous connective tissue progresses so as to stretch from the surface of each columnar member of the device for producing a tissue construct toward the inside of the hollow portion of the device, and the connective tissues formed around columnar members adjacent to each other become joined to each other, thereby causing a core portion having, on its outer surface, a recess that is recessed from the opening of the device toward the hollow portion of the device to be formed; and somatic stem cells derived from the living body are accumulated in the recess to form a somatic stem cell-accumulated tissue.

In addition, immunohistochemical staining was performed in the same way as in Example 3. In the connective tissue of the core portion, while many DAPI-stained cell nuclei were detected, mesenchymal stem cell marker CD90 expression was negative, and pluripotent stem cell marker SSEA-4 expression was also negative. In contrast, many CD90-positive somatic stem cells and many SSEA-4-positive somatic stem cells were detected in the somatic stem cell-accumulated tissue (the pocket portion).

When the device for producing a tissue construct was placed for 3 days after implantation in a living body, formation of the core portion having a recess on its outer surface was shown, but none of CD90-positive cells and SSEA-4-positive cells were detected in the somatic stem cell-accumulated tissue and in the core portion. When the device was placed for 10 days, 2 weeks, 17 days, or 3 weeks, CD90-positive cells and SSEA-4-positive cells were shown in the core portion.

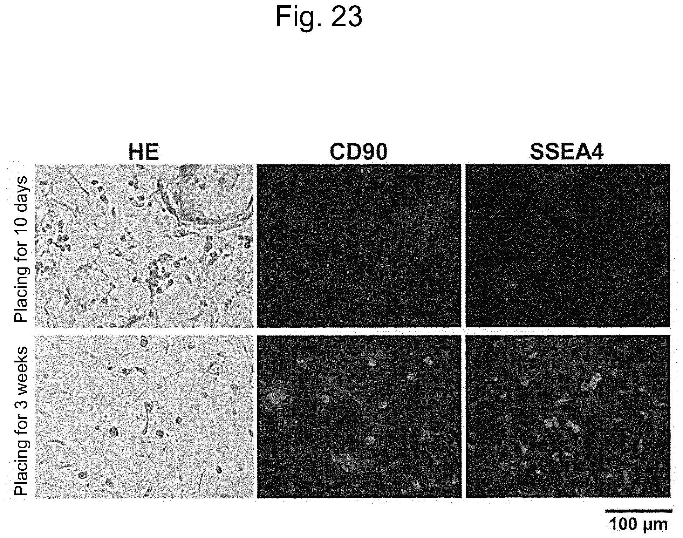

As shown in Fig. 23, the results of HE staining show that the somatic stem cell-accumulated tissue contained a lot of fibrin when the device was placed for 10 days. When the device was placed for 3 weeks, the amount of fibrin in the somatic stem cell-accumulated tissue was lower than that detected when the device was placed for 10 days. When the device was placed for 3 weeks, in contrast, the number of CD90-positive cells and the number of the SSEA-4-positive cells detected in the somatic stem cell-accumulated tissue were significantly higher compared to those detected when the device was placed for 10 days.

The number of CD90-positive cells in the somatic stem cell-accumulated tissue detected when the device was placed for 2 weeks was approximately intermediate between the number of the cells detected when the device was placed for 10 days and the number of cells detected when the device was placed for 3 weeks. The number of SSEA-4-positive cells in the somatic stem cell-accumulated tissue detected when the device was placed for 2 weeks was only somewhat higher than that detected when the device was placed for 10 days. This indicates that SSEA-4-positive cells in the somatic stem cell-accumulated tissue largely increased between 2 weeks and 3 weeks after the implantation in a living body.

The results above indicate that the number of somatic stem cells in the somatic stem cell-accumulated tissue increases depending on the duration during which the device for producing a tissue construct is placed in a living body.

### [Example 8]

Concerning the association between the structures of the devices for producing a tissue construct illustrated in Figs. 4 to 6 and the formation of the recess of the connective tissue and the somatic stem cell-accumulated tissue, the sections of the tissue construct produced by the placement for the duration of 3 weeks were subjected to fiber staining (HE staining) and examined for the association of the structures of the devices for producing a tissue construct illustrated in Figs. 4 to 6 with the formation of the recess of the connective tissue and the somatic stem cell-accumulated tissue, in the same way as in Example 7.

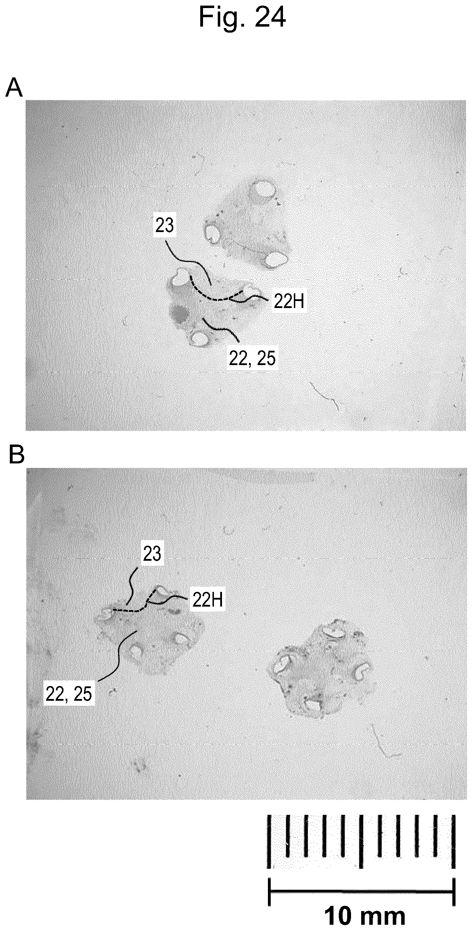

When the device for producing a tissue construct of the configuration illustrated in Fig. 4A was used to produce a tissue construct, the tissue construct produced had core portion 25 and somatic stem cell-accumulated tissue 23 formed in both of: (i) the hollow portion 10S of the device with the opening width W of 3.0 mm among three columnar members 13 and the depth D of 2.0 mm (the upper part of the device) and (ii) the hollow portion 10S of the device with the opening width W of 2.5 mm among four columnar members 13 and the depth D of 2.2 mm (the lower part of the device), the core portion 25 comprising fibrous connective tissue around each of the columnar members 13 and the supporting members 11A and 11B and in the center part, and having recesses 22H recessed from the openings toward the hollow portions of the device, the openings being surrounded (formed) by two adjacent columnar members 13, the supporting member 11A, and the supporting member 11B, and the somatic stem cell-accumulated tissue 23 being formed in the recesses (Fig. 24). The fibrous connective tissue 22 composing the core portion 25 stretched to the inside of the through-holes 11H of the 2 supporting members 11A of the device for producing a tissue construct. The tissue construct further had recesses 22H recessed from the through-holes (the supporting member openings) 11H toward the hollow portion of the device, and formation of somatic stem cell-accumulated tissue 23 was also observed in the recesses. The fibrous connective tissue formed in the hollow portion (i) and that formed in the hollow portion (ii) of the device were connected to each other by the fibrous connective tissue formed in the through-hole of the supporting member 11B.

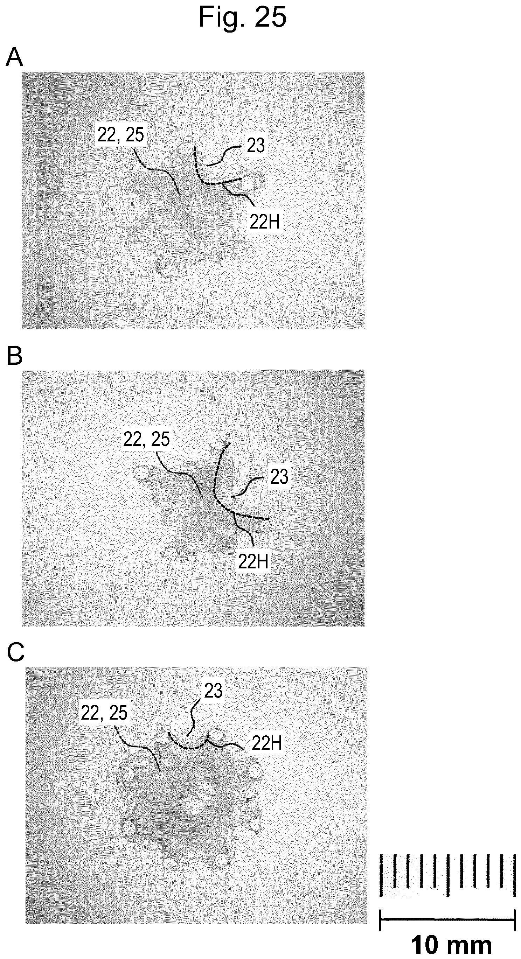

When the device for producing a tissue construct of the configuration illustrated in Fig. 4B was used to produce a tissue construct, the tissue construct produced had core portion 25 and somatic stem cell-accumulated tissue 23 formed in all of: (i) the hollow portion 10S of the device with the opening width W of 4.0 mm among six columnar members 13 and the depth D of 4.6 mm (the upper part of the device); (ii) the hollow portion 10S of the device with the opening width W of 6.0 mm among four columnar members 13 and the depth D of 4.0 mm (the middle part of the device); and (iii) the hollow portion 10S of the device with the opening width W of 3.0 mm among eight columnar members 13 and the depth D of 4.8 mm (the lower part of the device), the core portion 25 comprising fibrous connective tissue around each of the columnar members 13 and the supporting members 11A and 11B and in the center part, and having recesses 22H recessed from the openings toward the hollow portion of the device, the openings being surrounded (formed) by two adjacent columnar members 13 and two opposite supporting members (i.e., the supporting member 11A and the supporting member 11B; or two supporting members 11B), and the somatic stem cell-accumulated tissue 23 being formed in the recesses (Fig. 25). The fibrous connective tissue 22 composing the core portion 25 stretched to the inside of the through-holes 11H of the 2 supporting members 11A of the device for producing a tissue construct. The tissue construct further had recesses 22H recessed from the through-holes (the supporting member openings) 11H toward the hollow portion of the device, and formation of somatic stem cell-accumulated tissue 23 was also observed in the recesses. The fibrous connective tissue formed in the hollow portion (i), that formed in the hollow portion (ii), and that formed in the hollow portion (iii) of the device were connected to each other by the fibrous connective tissue formed in the through-holes of the 2 supporting members 11B.

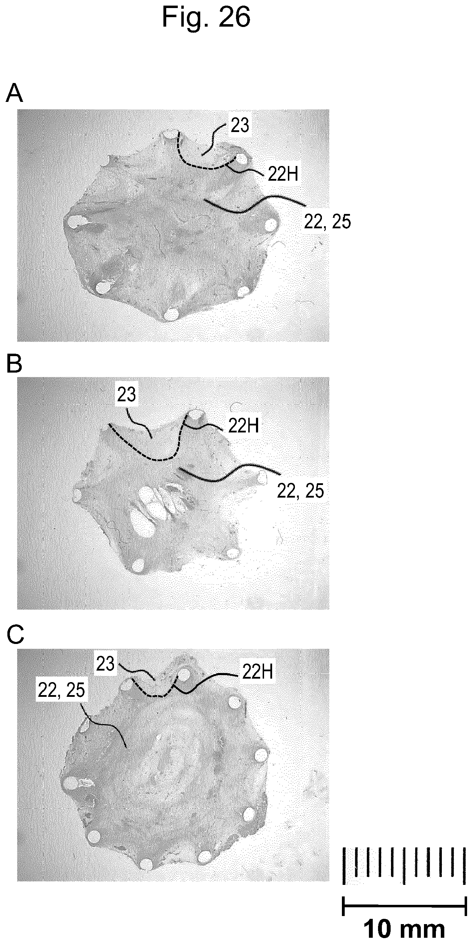

When the device for producing a tissue construct of the configuration illustrated in Fig. 4C was used to produce a tissue construct, the tissue construct produced had core portion 25 and somatic stem cell-accumulated tissue 23 formed in all of: (i) the hollow portion 10S of the device with the opening width W of 5.0 mm among eight columnar members 13 and the depth D of 7.1 mm (the upper part of the device); (ii) the hollow portion 10S of the device with the opening width W of 6.5 mm among six columnar members 13 and the depth D of 6.8 mm (the middle part of the device); and (iii) the hollow portion 10S of the device with the opening width W of 3.5 mm among ten columnar members 13 and the depth D of 7.3 mm (the lower part of the device), the core portion 25 comprising fibrous connective tissue around each of the columnar members 13 and the supporting members 11A and 11B and in the center part, and having recesses 22H recessed from the openings toward the hollow portion of the device, the openings being surrounded (formed) by two adjacent columnar members 13 and two opposite supporting members (i.e., the supporting member 11A and the supporting member 11B; or two supporting members 11B), and the somatic stem cell-accumulated tissue 23 being formed in the recesses (Fig. 26). The fibrous connective tissue 22 being a component of the core portion 25 stretched to the inside of the through-holes 11H of the 2 supporting members 11A of the device for producing a tissue construct. The tissue construct further had recesses 22H recessed from the through-holes (the supporting member openings) 11H toward the hollow portion of the device, and formation of somatic stem cell-accumulated tissue 23 was also observed in the recesses. The fibrous connective tissue formed in the hollow portion (i), that formed in the hollow portion (ii), and that formed in the hollow portion (iii) of the device were connected to each other by the fibrous connective tissue formed in the through-holes 11H of the 2 supporting members 11B.

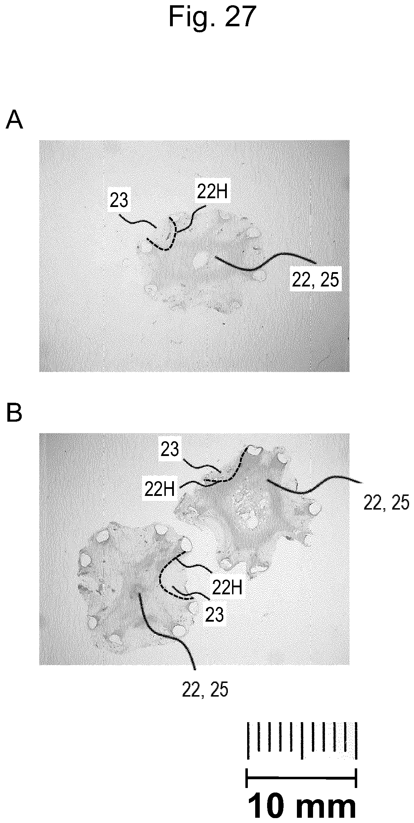

When the device for producing a tissue construct of the configuration illustrated in Fig. 5A was used to produce the tissue construct, the tissue construct produced had core portion 25 comprising fibrous connective tissue around each of the columnar members 13 and the supporting members 11A and in the center part, and having recesses 22H recessed from the openings toward the hollow portion of the device, the openings being surrounded (formed) by two adjacent columnar members 13 and two opposite supporting members 11A, and the somatic stem cell-accumulated tissue 23 being formed in the recesses. In each opening, formation of the recess 22H was observed within a range of the opening width W of 2.5 mm to 5.0 mm between the columnar members 13, and the somatic stem cell-accumulated tissue 23 was observed in the recess 22H. In a region with the opening width W of smaller than 2.5 mm, formation of the recess 22H was not observed. As an example, Fig. 27A shows an HE staining photograph of a section of the tissue construct as positioned approximately at the center of the tissue construct in the longitudinal direction. The recess 22H was observed at a site with the opening width W of 3.0 mm and the depth D of 4.8 mm, and the somatic stem cell-accumulated tissue 23 was also observed in the recess 22H. Fig. 27B shows an HE staining photograph of a section of the tissue construct as positioned near the end of the tissue construct in the longitudinal direction. At a site with the opening width W of 4.0 mm and the depth D of 4.6 mm, the recess 22H was observed, and the somatic stem cell-accumulated tissue 23 was also observed in the recess 22H; while at a site with the opening width W of 2.0 mm and the depth D of 4.9 mm, formation of the recess 22H was not observed (Fig. 27B). The fibrous connective tissue 22 of the core portion 25 stretched to the inside of the through-holes 11H of the 2 supporting members 11A of the device for producing a tissue construct. The tissue construct further had recesses 22H recessed from the through-holes (the supporting member openings) 11H toward the hollow portion of the device, and formation of somatic stem cell-accumulated tissue 23 was also observed in the recesses.

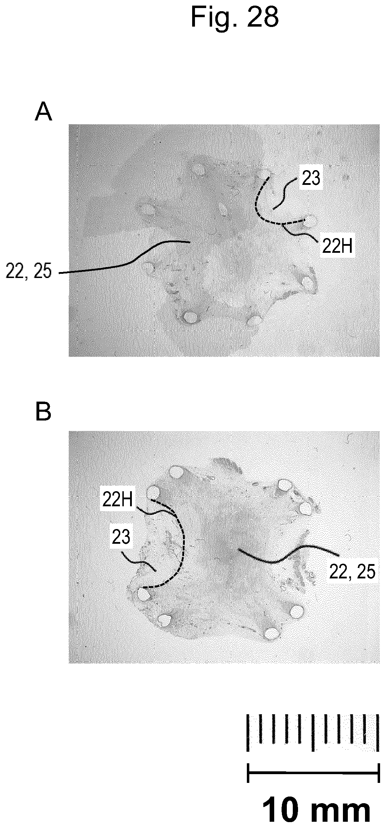

When the device for producing a tissue construct of the configuration illustrated in Fig. 5B was used to produce the tissue construct, the tissue construct produced had core portion 25 comprising fibrous connective tissue around each of the columnar members 13 and the supporting members 11A and in the center part, and having recesses 22H recessed from the openings toward the hollow portion of the device, the openings being surrounded (formed) by two adjacent columnar members 13 and two opposite supporting members 11A, and the somatic stem cell-accumulated tissue 23 being formed in the recesses. In each opening, formation of the recess 22H was observed within a range of the opening width W of 2.5 mm to 8.0 mm between the columnar members 13, and the somatic stem cell-accumulated tissue 23 was observed in the recess 22H. Within a range of the opening width W of smaller than 2.5 mm, formation of the recess 22H was not observed. As an example, Fig. 28A shows an HE staining photograph of a section of the tissue construct as positioned approximately at the center of the tissue construct in the longitudinal direction. The recess 22H was observed at a site with the opening width W of 4.0 mm and the depth D of 7.2 mm, and the somatic stem cell-accumulated tissue 23 was also observed in the recess 22H. Fig. 28B shows an HE staining photograph of a section of the tissue construct as positioned near the end of the tissue construct. At a site with the opening width W of 7.0 mm and the depth D of 6.6 mm, the recess 22H was observed, and the somatic stem cell-accumulated tissue 23 was also observed in the recess 22H; while at a site with the opening width W of 2.0 mm and the depth D of 7.4 mm, formation of the recess 22H was not observed (Fig. 28B). The fibrous connective tissue 22 of the core portion 25 stretched to the inside of the through-holes 11H of the 2 supporting members 11A of the device for producing a tissue construct. The tissue construct further had the recesses 22H recessed from the through-holes (the supporting member openings) 11H toward the hollow portion of the device, and formation of somatic stem cell-accumulated tissue 23 was also observed in the recesses.

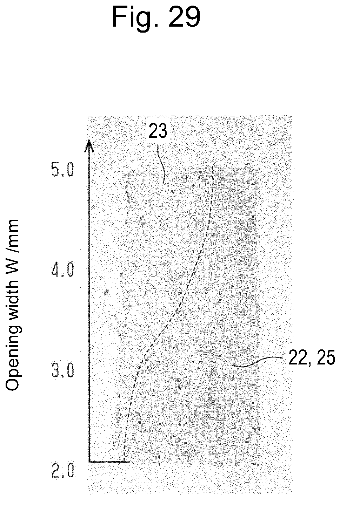

Fig. 29 shows an enlarged image of a photograph of an section having a cross section in the axial direction Ax of the tissue construct produced with the use of the device for producing a tissue construct illustrated in Fig. 5B, which had been subjected to fiber staining treatment. This image shows a structure of tissue formed directly below the opening with the opening width W continuously varying from 2.0 mm to 5.0 mm. As the opening width W increased in a range from 2.0 mm to 5.0 mm, as shown in Fig. 29, the depth of the recess 22H formed directly below the opening was found to increase. As the opening width W increased, also, the size (the volume) of the somatic stem cell-accumulated tissue 23 formed in the recess 22H directly below the opening was found to increase. In particular, the depth of the recess 22H was found to rapidly increase at the opening width W of 3.0 mm or more. It was thus considered that the size of the somatic stem cell-accumulated tissue 23 would rapidly increase at the opening width W of 3.0 mm or more.

When the device for producing a tissue construct of the configuration illustrated in Fig. 6A was used to produce the tissue construct, the tissue construct produced had core portion 25 comprising fibrous connective tissue around each of the columnar members 13, the large rectangular frame 11C, and the small rectangular frame 11D and in the center part, and having recesses recessed from the openings toward the hollow portion of the device, the openings being surrounded (formed) by two adjacent columnar members 13, the large rectangular frame 11C, and the small rectangular frame 11D, and the somatic stem cell-accumulated tissue 23 being formed in the recesses. When the device for producing a tissue construct of the configuration illustrated in Fig. 6B was used to produce the tissue construct, the tissue construct produced had core portion 25 comprising fibrous connective tissue around each of the columnar members 13, the large rectangular frame 11C, the small rectangular frame 11D, and the plate-like body 26, and in the center part, and having recesses 22H recessed from openings (i) and (ii) toward the hollow portion of the device, the openings (i) being surrounded (formed) by two adjacent columnar members 13, the large rectangular frame 11C, and the small rectangular frame 11D, and the opening (ii) being surrounded (formed) by a columnar member 13, the large rectangular frame 11C, the small rectangular frame 11D, and the plate-like body 26, and the somatic stem cell-accumulated tissue 23 being formed in the recesses. In an opening of the slit 26S (having the opening width of 1 mm) of the plate-like body 26, the recess was not formed. In each opening, formation of the recess 22H was observed within a range of the opening width W of 3.0 mm to 20.0 mm between the columnar members 13, and the somatic stem cell-accumulated tissue 23 was observed in the recess 22H. The device for producing a tissue construct with the configuration illustrated in Fig. 6A has the depth D ranging from 2.0 mm to 10.5 mm. The device for producing a tissue construct with the configuration illustrated in Fig. 6B, the bottom of the device being closed, has the depth D ranging from 2.0 mm to 20.5 mm because the deepest point of the hollow portion is located on the bottom. The fibrous connective tissue 22 of the core portion 25 stretched to the inside of the large rectangular frame 11C and the small rectangular frame 11D of the device for producing a tissue construct. The tissue construct further had the recesses 22H recessed from the openings of the large rectangular frame 11C and the small rectangular frame 11D (both being the supporting members) toward the hollow portion of the device, and formation of somatic stem cell-accumulated tissue 23 was also observed in the recesses.

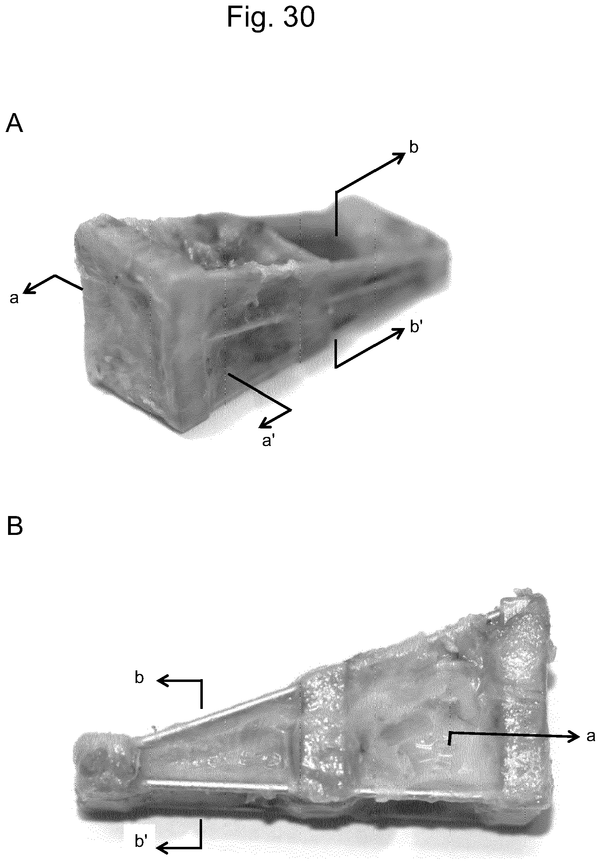

When the device for producing a tissue construct of the configuration illustrated in Fig. 6C was used to produce the tissue construct, the tissue construct produced had core portion 25 comprising fibrous connective tissue around each of the columnar members 13, the large rectangular frame 11C, the medium rectangular frame 11E, and the small rectangular frame 11D and in the center part, and having recesses 22H recessed from the openings (i) and (ii) toward the hollow portion of the device, the openings (i) being surrounded (formed) by two adjacent columnar members 13, the large rectangular frame 11C, and the medium rectangular frame 11E, and the openings (ii) being surrounded (formed) by two adjacent columnar members 13, the medium rectangular frame 11E, and the small rectangular frame 11D, and the somatic stem cell-accumulated tissue 23 being formed in the recesses (Fig. 30). In each opening, formation of the recess 22H was observed within a range of the opening width W of 3.0 mm to 20.0 mm between the columnar members 13, and the somatic stem cell-accumulated tissue 23 was observed in the recess 22H. The device for producing a tissue construct with the configuration illustrated in Fig. 6C has the depth D ranging from 2.0 mm to 10.5 mm. The fibrous connective tissue 22 of the core portion 25 stretched to the inside of the large rectangular frame 11C, the medium rectangular frame 11E, and the small rectangular frame 11D of the device for producing a tissue construct. The tissue construct further had the recesses 22H recessed from the openings of the large rectangular frame 11C and the small rectangular frame 11D (both being the supporting members) toward the hollow portion of the device, and formation of somatic stem cell-accumulated tissue 23 was also observed in the recesses.

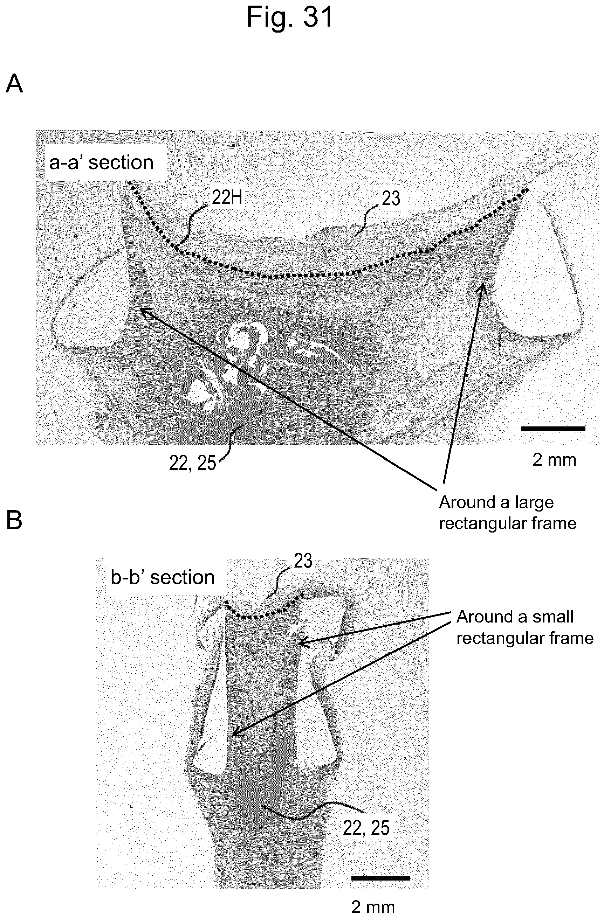

Fig. 31 shows the results of HE staining of a section of the tissue construct (Fig. 30) produced with the use of the device for producing a tissue construct with the configuration illustrated in Fig. 6C. The tissue construct was cut at positions indicated in Fig. 30. Fig. 31A shows a staining image of the somatic stem cell-accumulated tissue 23 formed directly below the opening of the large rectangular frame 11C, on a vertical cross section (a-a'). Fig. 31B shows a staining image of the somatic stem cell-accumulated tissue 23 formed directly below the opening of the small rectangular frame 11D, on a vertical cross section (transverse, b-b'). In all of the openings, formation of the recess 22H recessed toward the hollow portion of the device and somatic stem cell-accumulated tissue 23 in the recess was observed. In the fibrous connective tissue around the large rectangular frame 11C and the small rectangular frame 11D, dense collagen fibers were observed.

The Examples above show that formation of recess 22H was formed in the tissue construct within a range of the opening width W of 2.5 mm or larger of the device for producing a tissue construct. In contrast, formation of recess 22H was not observed directly below an opening with the opening width W of smaller than 2.5 mm. At the least, when the depth D or the maximal depth D was 2 mm or more, formation of the recess 22H was not affected.

It was shown that the invention using the device 10 for producing a tissue construct according to the present invention enables a tissue construct comprising the somatic stem cells 23C accumulated in the recess 22H and enriched in the somatic stem cells 23C to be produced in a shorter period of placement in a living body, such as within one month or even in approximately 2 to 3 weeks.

All publications, patents, and patent applications cited herein are incorporated herein by reference in their entirety.

### Description of Reference signs

Ax: axial direction; Ci: circumferential direction; Ct: center point, D: depth; S: circular cylindrical plane; W: opening width; 10: device for producing a tissue construct; 10F: opening edge; 10H: opening; 10S: hollow portion; 11: supporting member; 11A: first supporting member; 11B: second supporting member; 11H: through-hole; 12H: pass-through opening; 13: columnar member; 16: linear member; 21: body tissue; 22: fibrous connective tissue; 22C: fibroblast; 22H: recess; 23: somatic stem cell-accumulated tissue; 23C: somatic stem cell; 24: recess periphery; 25: core portion.

## Claims

1. A tissue construct comprising a core portion having a recess and composed of fibrous connective tissue, and loose fibrous, somatic stem cell-accumulated tissue comprising type III collagen and somatic stem cells which is formed in the recess.

2. The tissue construct according to Claim 1, wherein the core portion has one, or two or more recesses.

3. The tissue construct according to Claim 1 or 2, wherein the core portion has at least three recesses.

4. The tissue construct according to any one of Claims 1 to 3, wherein the recess has an opening width of at least 2.5 mm.

5. The tissue construct according to any one of Claims 1 to 4, wherein the core portion has a sectional width of at least 2.5 mm.

6. The tissue construct according to any one of Claims 1 to 5, wherein the somatic stem cells comprise at least either mesenchymal stem cells or pluripotent stem cells.

7. The tissue construct according to any one of Claims 1 to 6, wherein the tissue construct has a rod-like shape, and a plurality of the recesses are located along the circumferential direction.

8. The tissue construct according to any one of Claims 1 to 7, wherein the tissue construct has a rod-like shape, and a plurality of the recesses are located along the axial direction.

9. The tissue construct according to any one of Claims 1 to 6, wherein the tissue construct has a generally polyhedral shape, and a plurality of the recesses are located on different planes thereof.

10. The tissue construct according to any one of Claims 1 to 9, wherein the tissue construct has a non-tubular and non-sheet shape.

11. The tissue construct according to any one of Claims 1 to 10, which is formed by placing a device with a hollow portion for producing a tissue construct in an environment comprising biological tissue materials,
wherein the device for producing a tissue construct has a frame body that forms the hollow portion,
the frame body has an opening that communicates from the hollow portion to an external space of the device for producing a tissue construct, and
the frame body defines the shape of the tissue construct formed in the hollow portion.

12. The tissue construct according to Claim 11, wherein the tissue construct fills the hollow portion in the device for producing a tissue construct and the core portion is adhered to the surface of the frame body.

13. A method for collecting somatic stem cells comprising separating somatic stem cell-accumulated tissue or somatic stem cells from the tissue construct according to any one of Claims 1 to 12.

14. A device for producing a tissue construct comprising:
a hollow portion; and
a frame body that forms the hollow portion,
wherein the frame body has an opening that communicates from the hollow portion to an external space of the device for producing a tissue construct, and
the tissue construct comprises a core portion having a recess and composed of fibrous connective tissue, and loose fibrous, somatic stem cell-accumulated tissue comprising type III collagen and somatic stem cells which is formed in the recess.

15. The device for producing a tissue construct according to Claim 14, which is used to form a tissue construct comprising a core portion having a recess and composed of fibrous connective tissue, and loose fibrous, somatic stem cell-accumulated tissue comprising type III collagen and somatic stem cells which is formed in the recess by placing the device in an environment comprising body tissue materials,
wherein the device for producing a tissue construct comprises a hollow portion, and a frame body that forms the hollow portion,
the frame body has an opening that communicates from the hollow portion to an external space of the device for producing a tissue construct,
the frame body defines the shape of the tissue construct formed in the hollow portion, and
when the device is placed in the environment comprising body tissue materials, fibrous connective tissue is formed to stretch from the surface of the frame body toward the inside of the hollow portion, thereby causing the core portion having a recess recessed from the opening toward the hollow portion to be formed, and loose fibrous tissue where somatic stem cells are accumulated is formed in the recess.

16. The device for producing a tissue construct according to Claim 14 or 15, wherein the opening has an opening width of at least 2.5 mm.

17. The device for producing a tissue construct according to any one of Claims 14 to 16, wherein the opening has an opening configuration that enables a circle with a diameter of 2.5 mm or larger to be inscribed in the opening.

18. The device for producing a tissue construct according to any one of Claims 14 to 17, wherein the frame body is composed of a columnar member and a supporting member, and the columnar member is fixed by the supporting member to maintain the shape of the frame body.

19. The device for producing a tissue construct according to Claim 18, wherein a plurality of the columnar members are fixed to the supporting members at the both ends of the columnar members and arranged in a circumferential direction on a hypothetical cylindrical plane sandwiched between the supporting members.

20. The device for producing a tissue construct according to Claim 18 or 19, wherein the opening is formed by the columnar member and the supporting member, or by a plurality of the columnar members.

21. The device for producing a tissue construct according to any one of Claims 14 to 20, wherein the opening has a polygonal, rectangular, trapezoidal, round, circular, or elliptic opening edge.

22. The device for producing a tissue construct according to any one of Claims 18 to 21, wherein the supporting member has a further opening that communicates from the hollow portion to an external space of the device.

23. A method for producing a tissue construct comprising a core portion having a recess and composed of fibrous connective tissue, and loose fibrous, somatic stem cell-accumulated tissue comprising type III collagen and somatic stem cells which is formed in the recess,
wherein the method comprises placing the device for producing a tissue construct according to any one of Claims 14 to 22 in an environment comprising body tissue materials, such as in a living body or in an isolated body tissue.
